# EUROPEAN PATENT SPECIFICATION

(11) **EP 0 507 950 B1**
(45) Date of publication and mention of the grant of the patent: **08.04.1998**
(21) Application number: 91915148.0
(22) Date of filing: 30.08.1991
(51) Int. Cl.: C07D 493/04

(54) **PROCESS FOR PRODUCING DIACETALS**
VERFAHREN ZUR HERSTELLUNG VON DIACETALEN
PROCEDE DE PRODUCTION DE DIACETALS

(30) Priority: 31.08.1990 JP 231059/90
(43) Date of publication of application: 14.10.1992
(73) Proprietor: NEW JAPAN CHEMICAL CO.,LTD., Kyoto-shi Kyoto 612 (JP)
(72) Inventor: KOBAYASHI, Toshiaki, 11-16, Suzaku 5-chome, Nara 630 (JP); KITAGAWA, Sachio, Yawata-shi Kyoto 614 (JP)
(74) Representative: VOSSIUS & PARTNER
(86) International application number: JP9101162
(87) International publication number: WO9204352

(56) References cited:
- JP-A- 42 082
- JP-A- 67 285
- JP-A- 203 389
- ALDRICH CHEMICAL Co., Inc., 1994, p. 744

## Description

### TECHNICAL FIELD

The present invention relates to new useful processes for producing diacetals.

### BACKGROUND ART

The inventors of the present invention previously found that certain diacetals, such as dibenzylidenesorbitols, which are structurally characterized by aromatic nuclei substituted by ester or amido groups are not only of value as organic gelling agents but also of value as antistatic, anti-fogging, antifouling and/or other resin improving agents for various thermoplastic resins such as polyvinyl chloride, polyolefins, polystyrene, acrylic resins, etc. or as nucleating agents for crystalline resins such as polyolefins, polyesters and so on (Japanese Patent Application No. 2-5746).

The present inventors endeavored to develop a novel, commercially profitable production technology for producing such diacetal compounds having ester and/or amido groups under mild conditions in improved yield.

For the production of such compounds, the following alternative processes could be contemplated.
(1) In the first place, the inventors attempted to oxidize the methyl groups of 1,3:2,4-bis(methylbenzylidene)sorbitol to carboxyl groups and, then, esterify or amidate the latter with an alcohol or amine. However, the process was found to be inefficient in selectively oxidizing the side chains of the aromatic nuclei.
(2) The inventors' further research suggested another approach, viz. esterification or amidation of 1,3:2,4-bis(carboxybenzylidene)sorbitol with an alcohol or amine but because of the difficulty to control inter-molecular condensation in the course of esterification or amidation, the process was found to be low in reaction yield.
(3) Meanwhile, there is a prior art technology comprising condensing a formylbenzoic acid ester or a formylbenzoic acid amide with a polyhydric alcohol (Published Japanese Unexamined Patent Applications No. 1-203389 and No. 2-42082). This technology (3) is useful for the production of diacetals having lower alkyl ester residues or lower alkylcarbamoyl groups of about 1 to 4 carbon atoms. However, with respect to diacetals having ester residues or carbamoyl groups other than such lower (C₁-C₄)alkyl ester residues or lower alkyl carbamoyl groups, this technology is of limited commercial value for the production of such diacetals, since it is difficult to prepare a starting material formylbenzoic acid ester or formylbenzoic acid amide easily by the ordinary esterification or amidation reaction.

The object of the invention is, therefore, to provide a production process by which not only diacetals having lower alkyl ester residues or lower alkylcarbamoyl groups of about 1 to 4 carbon atoms but also diacetals having other ester residues or carbamoyl groups can be synthesized with advantage on a commercial scale.

### DISCLOSURE OF THE INVENTION

In the subsequent course of their research, the inventors took note of the following facts.
(a) It is difficult to convert the lower alkyl ester residue or lower alkylcarbamoyl group of a formylbenzoic acid lower alkyl ester or formylbenzoic acid lower alkyl amide to a different ester residue or carbamoyl group by way of ester interchange or esteramide interchange, with the formyl group being kept intact, because the reactivity is low.
(b) However, if, prior to said conversion of the lower alkyl ester residue to another ester residue or a carbamoyl group by said ester interchange or ester-amide interchange, the lower alkyl ester of formylbenzoic acid is converted to a diacetal using a lower aliphatic monohydric alcohol of 1 to 4 carbon atoms in the presence of an acid catalyst for quantitative protection of the formyl group, the desired ester interchange or ester-amide interchange reaction can readily proceed.
(c) Furthermore, the condensation reaction between the ester or amide thus obtained and a polyhydric alcohol such as sorbitol proceeds with markedly improved selectivity and reaction rate as compared with the conventional process (3) mentioned above, presumably because the liberated C₁₋₄ alcohol exerts a favorable effect on the reaction.
(d) Of the contemplated diacetals, diacetals having lower alkyl ester residues on the benzylidene groups, such as 1,3:2,4-bis(methoxycarbonylbenzylidene)sorbitol, etc., are subject to ester interchange or ester-amide interchange with various alcohols or amines.

Predicated on the above finding, the present invention provides a novel, commercially advantageous production process for said diacetals which comprises subjecting a formylbenzoic acid lower alkyl ester to ester interchange to replace the lower alkyl ester residue with a desired ester residue or to ester-amide interchange to convert said lower alkyl ester residue to a desired amide group.

Thus, in accordance with the production process of the invention, diacetals of the following general formula (I) are provided. 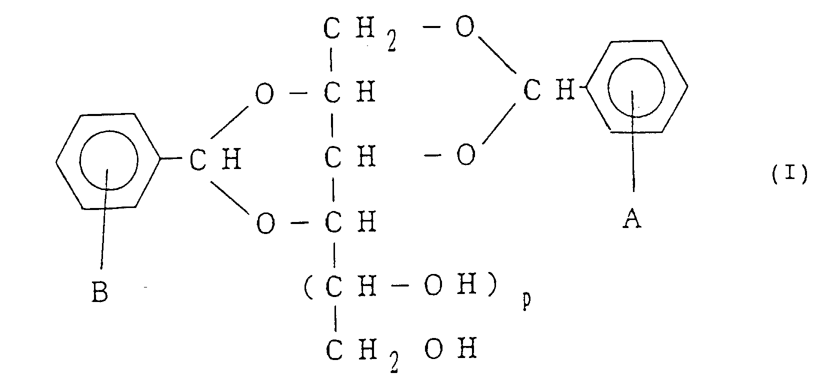 (wherein A and B are the same or different and each represents a hydrogen atom, -(Alk)ₜ (where Alk represents a lower alkyl group and t is equal to 1 through 3), -COOR¹ or -COOR²; or A and B are the same or different and each represents a hydrogen atom, -(Alk)ₜ (wherein Alk represents a lower alkyl group and t is equal to 1 through 3), -CONR³R^{3a} or -CONR⁴R^{4a},
wherein R¹ and R² are the same or different and each represents an alkyl group, a cycloalkyl group, a halogen-substituted alkyl group, an alkenyl group, an alkynyl group, an alkadienyl group, an alkapolyenyl group having 3 to 6 double bonds, an aryl group, an aralkyl group or a residue formed by removing one hydroxyl group from a monohydric or dihydric alcohol selected from the class consisting of polyoxyalkylene alkyl (or alkenyl) ethers, polyoxyalkylene alkylphenyl ethers, polyoxyalkylene alkyl esters, polyoxyalkylene-alkylamides, polyoxyalkylene benzoic acid esters, polyesters, ethylene oxide or polyethylene oxide adducts of primary or secondary amines, rosin alcohol, alkanediols containing 2 to 500 carbon atoms, polyethylene glycols, polypropylene glycols, ethylene oxide-propylene oxide copolymers, 2,2-bis(4-hydroxy-phenyl)propane and its lower alkylene oxide adducts and bis(4-hydroxyphenyl)methane and its lower alkylene oxide adducts;
R³ and R⁴ are the same or different and each represents an alkyl group, a cycloalkyl group, a halogen-substituted alkyl group, an alkenyl group, an alkynyl group, an alkadienyl group, an alkapolyenyl group containing 3 to 6 double bonds, an aryl group or an aralkyl group; R^{3a} and R^{4a} are the same or different and each represents a hydrogen atom or a group which is the same as R³ or R⁴, or
-NR³R^{3a} and -NR⁴R^{4a} are the same or different and each represents a residue formed by removing one hydrogen atom attached to one nitrogen atom of a primary or secondary mono- or diamine selected from the class consisting of N-(2-hydroxyethyl)-N-(C₁-C₃₀alkyl)amines, mono- and di-C₂-C₃ akanolamines, alkyldiamines containing 2 to 20 carbon atoms, aryldiamines containing 6 to 20 carbon atoms, aralkyldiamines containing 7 to 20 carbon atoms, and polyoxyethylenediamine (degree of polymerization of ethylene oxide = 1 - 100), polyoxypropylenediamine (degree of polymerization of propylene oxide = 1 - 100) and polyoxyethylene-polyoxypropylene-diamine (degree of polymerization = 1 - 100 for each of ethylene oxide and propylene oxide), which can be represented by the general formula

   H₂N(T¹O)ₙ(T²O)ₛ-T³-NH₂

   (wherein T¹ and T² are different and each represents an ethylene group (-CH₂CH₂-) or a propylene group (-CH₂CH(CH₃)-); T³ is the same as T¹ or T²; n and s are the same or different and each is equal to 0 through 100);
p is equal to 0 or 1;
with the proviso that both of A and B cannot concurrently be hydrogen atom or -(Alk)ₜ but at least one of A and B should represent either -COOR¹ or -COOR² or either -CONR³R^{3a} or -CONR⁴R^{4a}, and that at least one of R¹ and R² or at least one of R³, R^{3a}, R⁴ and R^{4a} should have at least one group selected among hydroxy group, amino group, ether group, ester group, amide group, halogen atom and rosin alcohol residue.)

Referring to the above general formula (I), the presence of said group selected from among hydroxy group, amino group, ether group, ester group, amide group, halogen atom and rosin alcohol residue at the chain terminus of at least one of substituents A and B or within the chain thereof insures a marked improvement in the resin improving function (improvement of antistatic, anti-fogging and antifouling functions).

In the diacetal compound of the above general formula (I), the position of the ester residue or carbamoyl group substituting the aromatic nucleus may be ortho, meta or para.

Throughout this specification and the appended claims, the term 'lower alkyl' represents a lower alkyl group containing 1 to 4 carbon atoms unless otherwise indicated, irrespective of whether it occurs independently or exists in other substituent group.

Referring to general formula (I), R¹ and R² each specifically represents an alkyl group (1 - 40 carbon atoms), a cycloalkyl group (5 - 10 carbon atoms), an alkyl group (1 - 30 carbon atoms) substituted by halogen, e.g. fluorine, an alkenyl group (3 - 40 carbon atoms), alkynyl group (3 - 40 carbon atoms), an alkadienyl group (4 - 40 carbon atoms), an alkapolyenyl group (3 - 6 double bonds, 6 - 24 carbon atoms), an aryl group (phenyl, naphthyl, etc.), an aralkyl group (7 - 40 carbon atoms) such as phenylalkyl, or a residue formed by removing one hydroxyl group from any of the following alcohols.
- Poly(degree of polymerization = 1 - 100)oxyalkylene alkyl(1 - 40 carbon atoms) or alkenyl(2 - 40 carbon atoms) ethers

   R'O(TO)ₙH

   [wherein R' represents an alkyl group of 1 to 40 carbon atoms or an alkenyl group of 2 to 40 carbon atoms; T represents a lower alkylene group of 2 to 3 carbon atoms; n is equal to 1 through 100]
- Poly(degree of polymerization = 1 - 100)oxyalkylene alkyl(1 - 40 carbon atoms)phenyl ethers, 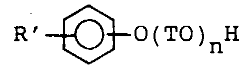 [wherein R' represents an alkyl group of 1 to 40 carbon atoms; T represents a lower alkylene group of 2 to 3 carbon atoms; n is equal to 1 through 100]
- Poly(degree of polymerization = 1 - 100)oxyalkylene alkyl(1 to 20 carbon atoms) esters

   R''COO(TO)ₙH

   [wherein R'' represents an alkyl group of 1 to 20 carbon atoms; T represents a lower alkylene group of 2 to 3 carbon atoms; n is equal to 1 through 100]
- N,N-bis[poly(degree of polymerization = 1 - 80)oxyalkylene]alkyl(1 to 30 carbon atoms)amides

   R^{a}CON[(TO)ₘH]₂

   [wherein R^{a} represents an alkyl group of 1 to 30 carbon atoms; T represents a lower alkylene group of 2 to 3 carbon atoms; m is equal to 1 through 80]
- Poly(degree of polymerization = 1 - 100)oxyalkylene benzoic acid esters

   C₆H₅COO(TO)ₙH

   [wherein T represents a lower alkylene group of 2 to 3 carbon atoms; n is equal to 1 through 100]
- Polyesters (Mn = 200 - 100,000)
- Poly(degree of polymerization = 1 - 100)oxyalkylene adducts of C₁₋₅₀ aliphatic or alicyclic primary or secondary amines

   (R^{b})₂N(TO)ₙH or R^{b}N[(TO)ₙH]₂

   [wherein R^{b} represents an alkyl group of 1 to 25 carbon atoms; T represents a lower alkylene group of 2 to 3 carbon atoms; n is equal to 1 through 100]
- Rosin alcohol
- Alkanediols of 2 to 500 carbon atoms
- Polyethylene glycol, polypropylene glycol and ethylene oxide-propylene oxide copolymer (degree of polymerization = ca. 2 - 800)
- 2,2-Bis(4-hydroxyphenyl)propane, bis(4-hydroxyphenyl)methane and lower alkylene oxide adducts thereof (the number of moles of lower alkylene oxide added= 1 - 20)
- Polyether monoalcohols and diols obtainable by ring-opening polymerization of tetrahydrofuran, dioxolane or the like.

Among examples of R³ and R⁴ are alkyl groups (1 to 40 carbon atoms), cycloalkyl groups (5 to 10 carbon atoms), halogen (e.g. fluorine)-substituted alkyl groups (1 to 30 carbon atoms), alkenyl groups (3 to 40 carbon atoms), alkynyl groups (3 to 40 carbon atoms), alkadienyl groups (4 to 40 carbon atoms), alkapolyenyl groups (3 to 6 double bonds, 6 to 24 carbon atoms), aryl groups (phenyl, naphthyl, etc.) and aralkyl groups (7 to 40 carbon atoms) such as phenylalkyl groups. Examples of R^{3a} and R^{4a} are hydrogen and the same groups as R³ and R⁴. Moreover, R³ and R^{3a} or R⁴ and R^{4a}, taken together with the adjacent nitrogen atom (that is to say in the form of -NR³R^{3a} or -NR⁴R^{4a}), represent a residue formed by removing one hydrogen atom attached to one nitrogen atom of a mono- or diamine such as those mentioned below.
- N-(2-Hydroxyethyl)-N-(C₁-C₃₀ alkyl)amines
- Mono- or di-C₂-C₃ alkanolamines, such as monoethanolamine, diethanolamine, monopropanolamine, dipropanolamine and so on.
- Alkyldiamines of 2 to 20 carbon atoms, such as ethylenediamine, hexamethylenediamine, decanediamine and so on.
- Aryldiamines of 6 to 20 carbon atoms, such as 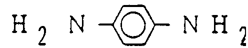 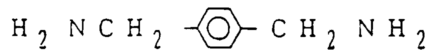 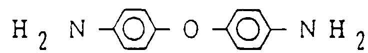 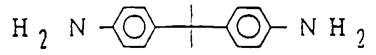 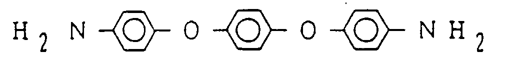
- Aralkyldiamines of 7 to 20 carbon atoms, such as 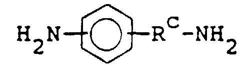 [wherein R^{c} represents an alkylene group of 1 to 14 carbon atoms] 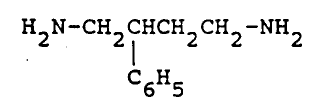
- Polyoxyalkylenediamines of the general formula

   H₂N(T¹O)ₙ(T²O)ₛ-T³-NH₂

   [wherein T¹ and T² may be the same or different and each represents an ethylene group (-CH₂CH₂-) or a propylene group (-CH₂CH(CH₃)-); T³ is the same as T¹ or T²; n and s are the same or different and each is equal to 0 - 100], such as polyoxyethylenediamine (degree of polymerization = 1 - 100), polyoxypropylenediamine (degree of polymerization = 1 - 100), polyoxyethylenepolyoxypropylenediamine (the degree of polymerization = 1 - 100 for each of ethylene oxide and propylene oxide) and so on.

Referring to the groups represented by R¹, R², R³, R^{3a}, R⁴ and R^{4a}, the oxyalkylene group includes ethylene oxide, propylene oxide and the corresponding co-adducts, and is preferably an ethylene oxide adduct or a propylene oxide-ethylene oxide co-adduct.

Regarding the diacetals of the above general formula (I), the compounds which are preferred from the standpoint of ease of production, etc. are shown below.

One group of diacetals of general formula (I) consists of compounds in which A and B are the same, e.g. both of A and B represent -COOR¹. In this connection, R¹ preferably contains at least one member of an ether group, a glycol ether group, a hydroxyl group and the like. Preferred examples of the group R¹ are residues obtained by removing one hydroxyl group from an alcohol such as an alkanediol of about 2 to 500, preferably about 2 to 30 carbon atoms, a poly(C₂-C₃)alkylene glycol, a polyoxyalkylene alkyl or alkenyl ether of the general formula

R'O(TO)ₙH

[wherein R' represents a C₁₋₄₀ alkyl or C₂₋₄₀ alkenyl group; T represents a lower (C₂₋₃) alkylene group; n is equal to 1 through 100], a polyoxyalkylene alkylphenyl ether of the general formula 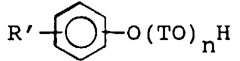 [wherein R' represents an alkyl group of 1 to 40 carbon atoms; T represents a lower alkylene group of 2 to 3 carbon atoms; n is equal to 1 through 100], or N,N-bis[polyoxyalkylene]alkylamide represented by the general formula

R^{a}CON[(TO)ₘH]₂

[wherein R^{a} represents an alkyl group of 1 to 30 carbon atoms; T represents a lower alkylene group of 2 to 3 carbon atoms; m is equal to 1 through 80], rosin alcohol, etc.

Another group of compounds of general formula (I) consists of compounds wherein A and B are the same and represent -CONR³R^{3a}. In this connection, the group -NR³R^{3a} is preferably a residue formed by removing one hydrogen atom attached to a nitrogen atom of a primary or secondary mono- or diamine containing ether group(s), glycol ether group(s), hydroxyl group(s) or other group(s). Particularly, the group -NR³R^{3a} is preferably a residue formed by removing one hydrogen atom attached to one nitrogen atom of a primary or secondary mono- or diamine selected from the class consisting of N-(2-hydroxyethyl)-N-(C₁-C₃₀ alkyl)amines, mono- or di-C₂-C₃ alkanolamines, alkyldiamines of 2 to 20 carbon atoms, aryldiamines of 6 to 20 carbon atoms, aralkyldiamines of 7 to 20 carbon atoms, and polyoxyethylenediamines (degree of polymerization of ethylene oxide = 1 - 100), polyoxypropylenediamines (degree of polymerization of propylene oxide = 1 - 100) and polyoxyethylene-polyoxypropylenediamines (degree of polymerization = 1 - 100 for each of ethylene oxide and propylene oxide), which can be represented by the general formula

H₂N(T¹O)ₙ(T²O)ₛ-T³-NH₂

[wherein T¹ and T² are different and each represents an ethylene group (-CH₂CH₂-) or a propylene group (-CH₂CH(CH₃)-); T³ is the same as T¹ or T²; n and s are the same or different and each is equal to 0 through 100]. Particularly preferred are compounds in which A and B are the same and both represent -CONR³R^{3a} (wherein the group -NR³R^{3a} represents a residue formed by removing one hydrogen atom attached to the nitrogen atom of an N-(2-hydroxyethyl)-N-(C₁-C₃₀ alkyl)amine or a mono- or di-C₂-C₃ alkanolamine).

A still another group of compounds of general formula (I) consists of compounds in which one of A and B is H or -(Alk)ₜ, with the other being -COOR¹ or -CONR³R^{3a}. In this connection, too, the group -COOR¹ or -CONR³R^{3a} is preferably one of the groups mentioned as preferred examples for the compounds of general formula (I) wherein A and B are the same.

One of the features of the production process for diacetals of general formula (I) according to the invention resides in the use of a di-lower alkyl acetal compound obtainable by converting the formyl group of a formylbenzoic acid derivative into dialkyl acetal with a lower alcohol. Another feature of the production process according to the invention resides in the fact that the di-lower alkyl acetal of a formylbenzoic acid lower alkyl ester reacts with an alcohol or an amine to consummate an ester interchange reaction or an ester-amide interchange reaction with good efficiency.

The embodiments of the process of the invention are shown in the following reaction schema-1.

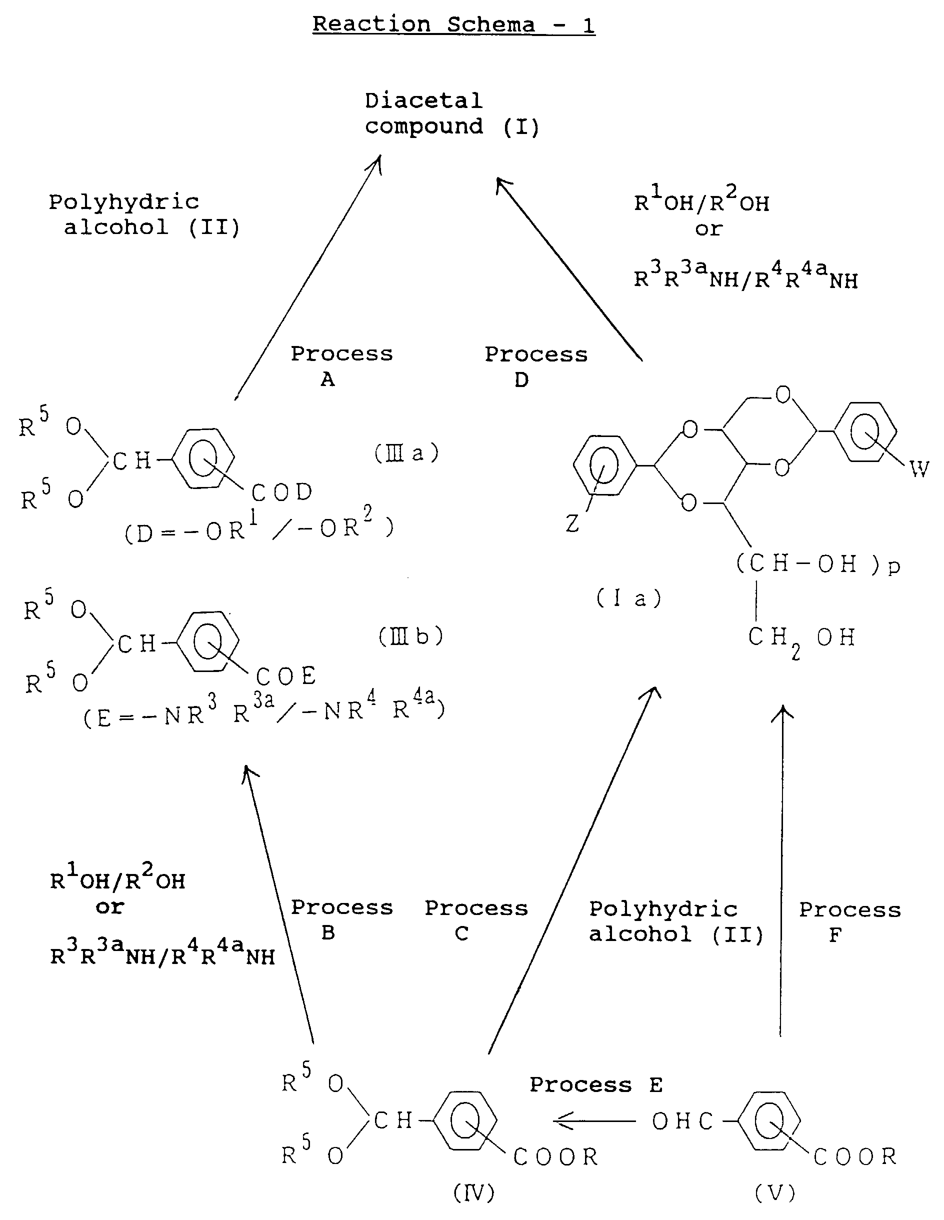

In the above reaction schema-1, R and R⁵ each represents a lower alkyl group of 1 to 4 carbon atoms and R¹ through R⁴, R^{3a}, R^{4a} and p are as defined hereinbefore for general formula (I). W and Z are the same or different and each represents H, -(Alk)ₜ (Alk represents a lower alkyl group; t is equal to 1 through 3) or -COOR (R is as defined above); provided, however, that W and Z cannot concurrently be hydrogen atom or -(Alk)ₜ.

### Process A

Process A is characterized in that a polyhydric alcohol of general formula (II), such as sorbitol, xylitol or the like, and a formylbenzoic acid ester di-lower alkyl acetal of general formula (IIIa) or a formylbenzoic acid amide di-lower alkyl acetal of general formula (IIIb) are subjected to condensation reaction in a water-soluble organic solvent-hydrophobic organic solvent system, where necessary in the presence of an acid catalyst.

Thus, when a di-lower alkyl acetal of general formula (IIIa) or (IIIb) is used, the condensation reaction with a polyhydric alcohol (II) proceeds with great advantage so that a compound of general formula (I) wherein at least one of A and B represents either -COOR¹ or -COOR² , or either -CONR³R^{3a} or -CONR⁴R^{4a}, and at least one of R¹ and R² or at least one of R³ and R^{3a} or R⁴ and R^{4a} contains at least one kind of group selected from the class consisting of hydroxy group, amino group, ether group, ester group, amide group, halogen atom and rosin alcohol residue can be produced with particular advantage.

In the description of this Process A and other processes, each starting compound is selected so that at least one of the substituent groups A and B of the compound of general formula (I) will contain at least one kind of groups such as those mentioned above.

In Process A, a compound of general formula (I) wherein either one of A and B is hydrogen or -(Alk)ₜ can be obtained by subjecting said compound (IIIa) or (IIIb) and either benzaldehyde or a di-lower alkyl acetal thereof or benzaldehyde substituted by 1 to 3 lower alkyl groups or a di-lower alkyl acetal thereof, either in an optional order or as a mixture, to condensation reaction with a polyhydric alcohol (II).

Thus, Process A is a process for producing a compound of general formula (I) characterized by subjecting at least one ester of general formula (IIIa) 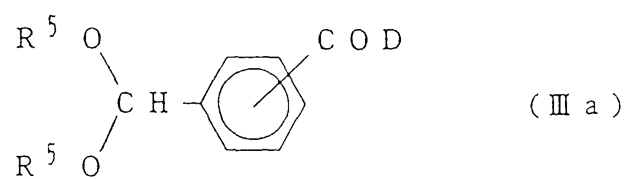 [wherein D represents -OR¹ or -OR² (R¹ and R² are as defined hereinbefore); R⁵ represents a lower alkyl group of 1 to 4 carbon atoms] or at least one amide of general formula (IIIb) 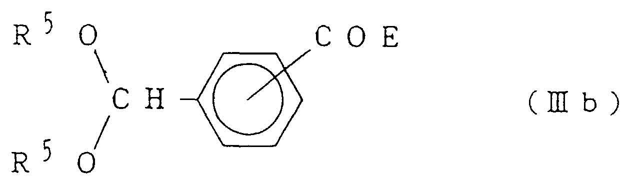 [wherein E represents -NR³R^{3a} or -NR⁴R^{4a} (R³, R^{3a}, R⁴ and R^{4a} are as defined hereinbefore); R⁵ is the same as above], or (a) an ester of general formula (IIIa) or an amide of general formula (IIIb) and (b) benzaldehyde or a di-lower alkyl acetal thereof or benzaldehyde substituted by 1 to 3 lower alkyl groups or a di-lower alkyl acetal thereof, either in an optional order or as a mixture, to condensation reaction with a polyhydric alcohol of general formula (II) 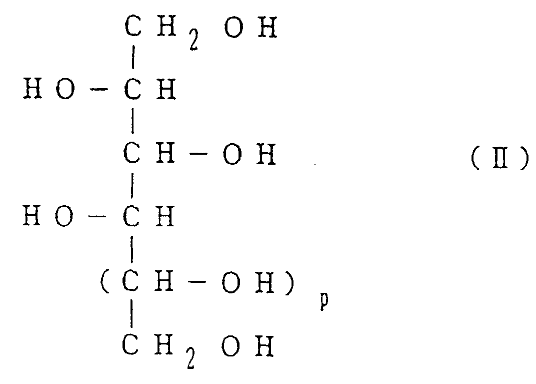 [wherein p is equal to 0 or 1] in a hydrophobic organic solvent and a water-soluble organic solvent, where necessary in the presence of an acid catalyst.

Process A can be conducted, for example in the following manner. A suitable reactor is charged with a polyhydric alcohol of general formula (II), a formylbenzoic acid ester dialkyl acetal of general formula (IIIa) or a formylbenzoic acid amide dialkyl acetal of general formula (IIIb), a water-soluble organic solvent and a hydrophobic organic solvent, and where necessary an acid catalyst, and the condensation reaction is conducted with heating at about 20 to 200°C, preferably about 40 to 130°C, and with stirring for about 1 to 20 hours, while removing the thus formed water, if so desired, with use of, for example, a Dean-Stark apparatus. In this manner, the desired compound of general formula (I) can be obtained in high yield.

The amount of the formylbenzoic acid ester dialkyl acetal of general formula (IIIa) or formylbenzoic acid amide dialkyl acetal of general formula (IIIb) to be used is generally about 1 to 10 moles and preferably about 1.5 to 5 moles, per mole of the polyhydric alcohol of general formula (II).

In the case where (a) said ester of general formula (IIIa) or amide of general formula (IIIb) and (b) benzaldehyde or a di-lower alkyl acetal thereof or benzaldehyde substituted by 1 to 3 lower alkyl groups or a di-lower alkyl acetal thereof are reacted, either in an optional order or as a mixture, with the polyhydric alcohol of general formula (II), the molar ratio reactant (a):reactant (b) can be optionally selected but generally is preferably adjusted to (a):(b) = about 1:3 through 3:1. In the case where the reactants (a) and (b) are fed independently in an optional order rather than as a mixture, the desired diacetal is preferably prepared by reacting, relative to one mole of the polyhydric alcohol of the formula (II), one of reactants (a) and (b), in an amount of about 0.2 to 4 moles, preferably about 0.5 to 2 moles, to give a monoacetal in the first place, and then reacting the other of reactants (a) and (b), in an amount of about 0.2 to 4 moles, preferably about 0.5 to 2 moles, in the second place.

The hydrophobic organic solvent mentioned hereinbefore includes, among others, cyclohexane and lower alkyl-substituted cyclohexanes, e.g. methylcyclohexane, ethylcyclohexane, etc.; chain hydrocarbons of 6 to 16 carbon atoms, e.g. n-hexane, kerosene, heptane, octane, decane, etc.; aromatic hydrocarbons such as benzene, toluene, xylene, etc.; halogenated hydrocarbons such as chloroform, chlorobenzene, etc.; di-lower alkyl ethers such as isopropyl ether, isoamyl ether, methyl butyl ether, etc.; nitro compounds such as nitromethane, nitrobenzene, etc.; and various esters such as methyl benzoate, butyl benzoate and so on. These solvents may be used alone or in combination. Mixtures of such solvents with water may also be employed. The amount of such solvent to be used is generally about 0.5 to 20 times (by weight), preferably about 1 to 15 times, based on the formylbenzoic acid ester dialkyl acetal of general formula (IIIa) or formylbenzoic acid amide dialkyl acetal of general formula (IIIb) or a combination of said reactants (a) and (b).

The water-soluble organic solvent mentioned hereinbefore includes, among others, N,N-dimethylformamide, dimethyl sulfoxide, dioxane, sulfolane and lower alcohols such as methanol, ethanol, propanol, butanol and so on. The amount of such solvent to be used is generally about 0.5 to 50% by weight and preferably about 1 to 30% by weight of the above hydrophobic organic solvent.

The acid catalyst which is used if so desired includes, among others, hydrochloric acid, sulfuric acid, phosphoric acid, zinc chloride, alkyl(C₂-C₁₂)benzene-sulfonic acids, G acid, L acid and so on. The catalyst is generally used in an amount of about 0.01 to 30 mole %, preferably about 0.1 to 15 mole %, based on the polyhydric alcohol of general formula (II).

### Process B

As mentioned hereinbefore, the formylbenzoic acid ester dialkyl acetal of general formula (IIIa) and the formylbenzoic acid amide dialkyl acetal of general formula (IIIb) can each be produced with great advantage by subjecting a formylbenzoic acid lower alkyl ester di-lower alkyl acetal [compound of general formula (IV)], as the starting material, to ester interchange reaction or amide-ester interchange reaction.

Thus, when a compound of general formula (IV) is subjected to the ester interchange or ester-amide interchange reaction with at least one of mono- or di-hydric alcohols of the general formulas

R¹OH and R²OH

[wherein R¹ and R² are as defined hereinbefore; provided that R¹ and R² are different from R] or at least one of mono- or diamines of the general formulas

R³R^{3a}NH and R⁴R^{4a}NH

[wherein R³, R^{3a}, R⁴ and R^{4a} are as defined hereinbefore], there is obtained an ester compound of general formula (IIIa) wherein R¹ and R² are different from R or an amide compound of general formula (IIIb).

The alcohol for use in the above ester interchange reaction may be a secondary alcohol or a tertiary alcohol but a mono- or diol containing at least one primary alcoholic hydroxyl group is preferred from the standpoint of reactivity. As preferred examples of such alcohol, there may be mentioned aliphatic alcohols containing 2 to 40 carbon atoms, such as ethanol, propanol, butanol, octanol, octadecanol, etc.; alkanediols containing about 2 to 500, preferably about 2 to 30 carbon atoms, such as ethylene glycol, propanediol, butanediol, pentanediol, hexanediol, decanediol, polyethylene diol, polybutadiene diol, hydrogenated polybutadiene diol, etc.; poly(C₂-C₃)alkylene glycols such as polyethylene glycol, polypropylene oxide-polyethylene oxide copolymer, etc.; 2,2-bis(4-hydroxyphenyl)propane, bis(4-hydroxyphenyl)methane and the (C₂-C₃)alkylene oxide adducts thereof; and various alcohols such as cycloalkyl alcohols (C₅-C₁₀), halogen(e.g. fluorine)-substituted alkyl alcohols (C₁-C₃₀), alkenyl alcohols (C₃-C₄₀), alkynyl alcohols (C₃-C₄₀), alkadienyl alcohols (C₄-C₄₀), alkapolyenyl alcohols (number of double bonds = 3 - 6, C₆-C₂₄), aryl alcohols, aralkyl alcohols (C₇-C₄₀) and poly(degree of polymerization = 1 - 100)oxyalkylene alkyl(C₁-C₄₀) or alkenyl (C₂-C₄₀) ethers, poly(degree of polymerization = 1 - 100)oxyalkylene alkyl(C₁-C₄₀)phenyl ethers, poly(degree of polymerization = 1 - 100)oxyalkylene alkyl (C₁-C₂₀) esters, N,N-bis poly(degree of polymerization = 1 - 80)-oxyalkylene]alkyl(C₁-C₃₀)amides, poly(degree of polymerization = 1 - 100)oxyalkylene benzoic esters, etc., polyesters (Mn = 200 - 100,000), polyether monoalcohols and diols which are obtainable by ring-opening polymerization of tetrahydrofuran, dioxane or the like, aliphatic or alicylic primary or secondary amine-poly(degree of polymerization = 1 - 100)oxyalkylene adducts, and rosin alcohols.

Among these alcohols, alkanediols of 2 to 500 carbon atoms, poly(C₂-C₃)alkylene glycols, poly(degree of polymerization = 1 - 100)oxyalkylene alkyl(C₁-C₄₀) or alkenyl(C₂-C₄₀) ethers, poly(degree of polymerization = 1 - 100)oxyalkylene alkyl(C₁-C₄₀)phenyl ethers, N,N-bis-[poly(degree of polymerization = 1 - 80)oxyalkylene]-alkyl(C₁-C₃₀)amides, etc. are preferred.

The amine for use in the above ester-amide interchange reaction is a mono- or diamine having at least one primary or secondary amino group. Among the primary or secondary monoamines suitable for the purpose are alkylamines containing 1 to 40 carbon atoms, cycloalkylamines of 5 to 10 carbon atoms, haloalkylamines of 1 to 30 carbon atoms, alkenylamines of 3 to 40 carbon atoms, alkynylamines of 3 to 40 carbon atoms, alkadienylamines of 4 to 40 carbon atoms, alkapolyenylamines of 6 to 24 carbon atoms, arylamines of 6 to 10 carbon atoms, aralkylamines of 7 to 40 carbon atoms, polyoxyethylenediamine, polyoxypropylenediamine, polyoxyethylene-polyoxypropylene-diamine, N-(2-hydroxyethyl)-N-C₁-C₃₀alkylamines, mono- and dialkanolamines and so on. Thus, there may be mentioned methylamine, N,N-dimethylamine, ethylamine, N,N-diethylamine, propylamine, N,N-dipropylamine, decylamine, N,N-didecylamine, stearylamine, N,N-distearylamine, cyclohexylamine, N,N-dicyclohexylamine, monoethanolamine, diethanolamine, aniline, N,N-diphenylamine, N-hydroxyethyl-N-decylamine, N-hydroxyethyl-N-stearylamine and so on. Among the diamines are alkyldiamines of 2 to 20 carbon atoms, aryldiamines of 6 to 20 carbon atoms, aralkyldiamines of 7 to 20 carbon atoms, polyoxyethylenediamine (degree of polymerization = 1 - 100), polyoxypropylenediamine (degree of polymerization = 1 - 100), polyoxyethylene-polyoxypropylene-diamine (degree of polymerization = 1 - 100, for each of ethylene oxide and propylene oxide). Specifically, there may be mentioned 1,3-propanediamine, 1,4-butanediamine, 1,6-hexamethylenediamine, p-phenylenediamine, xylenediamine, polyoxyethylenediamine, polypropylenediamine, N-(2-hydroxyethyl)ethylenediamine and so on.

Among the above-mentioned amines, preferred are N-(2-hydroxyethyl)-N-C₁-C₃₀alkylamines, di(C₂-C₃)alkanolamines, mono- and alkyldiamines of 2 to 20 carbon atoms, aryldiamines of 6 to 20 carbon atoms, aralkyldiamines of 7 to 20 carbon atoms, polyoxyethylenediamine (degree of polymerization = 1 - 100), polyoxypropylenediamine (degree of polymerization = 1 - 100) and polyoxyethylene-polyoxypropylene-diamine (degree of polymerization = 1 - 100 for each of ethylene oxide and propylene oxide).

The ester interchange or ester-amide interchange reaction is conducted in a solvent in the presence of a basic catalyst. The monohydric or dihydric alcohol or the amine is used in an amount of about 0.8 to 10 moles, or even more in excess, per mole of the compound of general formula (IV). Suitable examples of the basic catalyst are alkali metals such as lithium, sodium, potassium, etc., the corresponding hydrides, hydroxides, alcoholates thereof (e.g. sodium methylate, sodium ethylate, potassium tert-butylate, etc.) and alkali metal amides (e.g. NaNH₂, LiN(iso-C₃H₇)₂) and so on. The amount of the catalyst is about 0.01 to 10 moles per mole of the dialkyl acetal of general formula (IV).

The reaction solvent may be any solvent that is capable of dissolving the dialkyl acetal of general formula (IV) and inert to the reaction. Thus, there may be mentioned ether solvents such as tetrahydrofuran, dioxane, etc. and various other compounds such as dimethyl sulfoxide, dimethylformamide and so on. Furthermore, when said mono- or dihydric alcohol (R¹OH or R²OH) is a lower alcohol or said mono- or diamine (R³R^{3a}NH and R⁴R^{4a}NH) is a lower amine, such lower alcohol or lower amine may be used in excess so that it may be used as the reactant and the solvent.

The reaction temperature is generally about 0 to 100°C and preferably about 20 to 80°C. As the reaction system is stirred under such conditions for about 3 to 12 hours, there is obtained the desired formylbenzoic ester dialkyl acetal of general formula (IIIa) or the formylbenzoic acid amide dialkyl acetal of general formula (IIIb).

The above desired compounds can also be synthesized in the presence of an acid catalyst such as hydrochloric acid, sulfuric acid, phosphoric acid, toluenesulfonic acid, titanium tetraisopropoxide or the like. However, the use of the basic catalyst is preferred from the standpoints of reaction time and reaction yield.

The compound of general formula (IV) for use as the starting material for the above-described process B can be easily prepared by Process E as described below.

### Process E

In this process, a lower alkyl ester of formylbenzoic acid of the following general formula 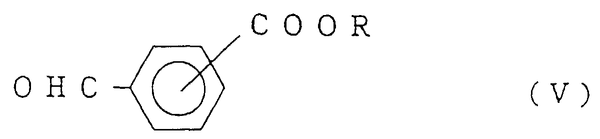 [wherein R represents a lower alkyl group of 1 to 4 carbon atoms] is converted to the acetal of general formula (IV) using a lower saturated aliphatic alcohol containing 1 to 4 carbon atoms (R⁵OH).

The lower alkyl ester of formylbenzoic acid of general formula (V), such as methyl p-formylbenzoate, can be prepared by the known methods and may also be obtained as a byproduct of terephthalic acid production. Using about 1 to 20 moles of said lower alcohol containing 1 to 4 carbon atoms per mole of such compound of general formula (V), the reaction is carried out in the presence of an acid catalyst, such as hydrochloric acid, sulfuric acid, phosphoric acid, toluenesulfonic acid, camphorsulfonic acid, oxalic acid, an acidic ion exchange resin, etc., at 20 to 100°C with stirring for about 1 to 12 hours, whereby the dialkyl acetal of general formula (IV) is obtained in almost quantitative yield. The amount of the acid catalyst is about 0.1 to 20 mole % based on the compound of general formula (V).

When the reaction is conducted using an excess of said lower alcohol, the alcohol functions as a reaction solvent as well.

If desired, a compound inert to the reaction can be used as the solvent. Among suitable examples of the solvent are ethers such as tetrahydrofuran, dioxane, etc.; benzene series hydrocarbons such as benzene, toluene, xylene, etc.; halogenated hydrocarbons such as chloroform, chlorobenzene, etc.; and alicyclic hydrocarbons such as cyclohexane, cycloheptane, etc., among others.

The above dialkyl acetal can also be produced by using a lower alcohol ester of orthoformic acid in lieu of said lower saturated aliphatic alcohol of 1 to 4 carbon atoms (R⁵OH).

Thus, the above series of processes A and B according to the invention comprises first reacting a compound of general formula (IV) with said mono- or dihydric alcohol or said amine and then reacting a polyhydric alcohol of general formula (II). However, the desired compound of general formula (I) can also be successfully produced by the reversed sequence shown as Process C and Process D in Reaction Schema-1, namely first reacting a compound of general formula (IV) with a polyhydric alcohol of general formula (II) and, then, reacting said mono- or dihydric alcohol or amine.

### Process C and Process F

Process C or F comprises subjecting at least one compound of general formula (IV) or at least one compound of general formula (V), or (a) a compound of general formula (IV) or a compound of general formula (V) and (b) benzaldehyde or a di-lower alkyl acetal thereof or benzaldehyde substituted by 1 to 3 lower alkyl groups or a di-lower alkyl acetal thereof, either in an optional order or as a mixture, to condensation reaction with a polyhydric alcohol of general formula (II) in a hydrophobic organic solvent and a water-soluble organic solvent, where necessary in the presence of an acid catalyst, to give a compound of general formula (Ia) wherein W=Z=-COOR (i.e. lower (C₁-C₄)alkyl esters, such as 1,3:2,4-bis(methoxycarbonylbenzylidene)sorbitol) or a compound wherein W is different from Z and one of them is -COOR with the other being -(Alk)ₜ (Alk represents a lower alkyl group; t is equal to 1 through 3).

This reaction can be carried out in the same manner as described hereinbefore for Process A. Thus, the amount of the compound of general formula (IV) or (V) to be used is about 1 to 10 moles, preferably about 1.6 to 5 moles, per mole of the polyhydric alcohol of general formula (II).

When (a) the compound of general formula (IV) or the compound of general formula (V) and (b) benzaldehyde or a di-lower alkyl acetal or benzaldehyde substituted by 1 to 3 lower alkyl groups or a di-lower alkyl acetal thereof are reacted in an optional order or as a mixture, the molar ratio reactant (a):reactant (b) can be freely selected but generally is preferably adjusted to (a):(b) = about 1:3 through 3:1. When the reactants (a) and (b) are fed independently in an optional order, not as a mixture, it is preferable that relative to 1 mole of the polyhydric alcohol of the formula (II), one of the reactants (a) and (b) be first reacted in an amount of about 0.2 to 4 moles, preferably about 0.5 to 2 moles, and, then, the other of (a) and (b) be reacted in an amount of about 0.2 to 4 moles, preferably about 0.5 to 2 moles.

The hydrophobic organic solvent, water-soluble organic solvent, acid catalyst and other conditions of reaction are substantially the same as those described for Process A.

### Process D

The compound of general formula (Ia) obtained in the above Process C or F is subjected to ester interchange or ester-amide interchange reaction with a mono-or dihydric alcohol (R¹OH, R²OH) or a mono- or diamine (R³R^{3a}NH, R⁴R^{4a}NH), whereby the desired diacetal compound (I) is obtained.

This reaction is carried out using about 1 to 7 moles of said mono- or dihydric alcohol or said amine and about 0.01 to 10 moles of a basic catalyst, each per mole of the compound of general formula (Ia) in the presence or absence of a solvent at 30 to 200°C, with stirring at atmospheric or subatmospheric pressure for 1 to 10 hours. The basic catalyst and solvent may be those mentioned for Process B.

In accordance with this process, the contemplated interchange reaction proceeds without intermolecular condensation and esterification. Therefore, this process is more advantageous than Process A particularly for the interchange reaction with said mono- or dihydric alcohol or amine having a molecular weight greater than about 100.

The respective products obtained in the above processes can be easily purified by the conventional isolation and purification procedures such as recrystallization, solvent extraction, supercritical fluid extraction and so on.

The diacetals obtainable as above are not only of value as gelling agents for fluid systems such as spilled oil, adhesives, perfumes, pharmaceuticals, polymers, etc. but also, because of their thixotropic property, of value as flowability-modifying agents for FRP, paints, ink, adhesives and other products. Furthermore, like the hithereto-known diacetals, the diacetals of the invention are useful as nucleating agents for crystalline resins such as polyolefins, polyethylene terephthalate and so on. To be specific, the use of the above low-melting compounds as nucleating agents is effective in reducing the molding temperature, while the diacetals having an oxyethylene ether chain, terminal hydroxyl group or an amino side chain exhibit antistatic and/or anti-fogging properties. The compounds of the invention which have halogen-substituted alkyl chains impart fouling resistance to molded resins.

### Examples

The following production example and examples are further illustrative of the invention.

Referring to the production example, working examples and comparative examples which appear below, the identification of product compounds of general formula (I) was generally carried out as follows.

Thus, in the case of compounds wherein the molecular weight of substituent A and/or B is comparatively small, viz. not greater than about 100, the product compound (I) was trimethylsilylated and the molecular weight was determined from the parent peak in the gas-mass spectrum (GCMS). NMR spectroscopy was also performed and from the formal proton in the vicinity of 5.6 ppm and its area, a two six-membered cyclic acetal structure shown in general formula (I) was confirmed. Furthermore, the existence of the hydroxy, amino, ether, ester or amide group was ascertained from the characteristic absorptions in the Fourier-transform infrared absorption spectrum (FTIR). Moreover, because the reaction with sodium metaperiodate showed an equimolar consumption of sodium metaperiodate relative to product (I), the existence of one vicinal diol was confirmed. After the oxidation reaction with sodium metaperiodate, the reaction product was reduced and further hydrolyzed. Because the resulting alditol was xylitol, the product (I) was found to have a 1,3:2,4-derivative structure.

In the case of compounds wherein the molecular weight of substituent A and B is comparatively large, viz. more than about 100, the molecular weight was not directly determined but the compound was first refluxed in the presence of methanol and alkali for about 2 hours to convert the substituent A and/or B to -COOCH₃. Then, the kind and number of moles of the liberated initial substituent group were confirmed to be pertinent by the terminal group assay (hydroxyl value or amine value) method. On the other hand, the compound wherein A and/or B is -COOCH₃ was identified in the same manner as the compounds wherein said substituent A and/or B has a comparatively small molecular weight.

### Production Example 1

### (1) Diacetalization

A 200 ml four-necked flask equipped with a stirrer, decanter-condenser, thermometer and gas feed piping was charged with 16.4 g (0.1 mole) of methyl p-formylbenzoate, 50 ml of methanol and 0.5 g of p-toluenesulfonic acid monohydrate and the mixture was stirred in a nitrogen gas atmosphere at 50°C for 1 hour. After cooling to room temperature, the reaction mixture was neutralized with a solution of sodium hydroxide in methanol and the methanol was then distilled off under reduced pressure. The reaction product was extracted with chloroform and the extract was washed with water. Removal of chloroform by distillation gave the desired methyl p-formylbenzoate dimethyl acetal in quantitative yield.

### (2) Ester interchange reaction

Then, a 300 ml flask was charged with 21 g (0.1 mole) of methyl p-formylbenzoate dimethyl acetal, 16.5 g (0.14 mole) of 2-butoxyethanol, 100 ml of dry tetrahydrofuran and 1.05 g of sodium methylate and the mixture was stirred at 65°C for 2.5 hours.

### (3) Isolation and purification

After cooling to room temperature, the reaction mixture was neutralized with a saturated aqueous solution of ammonium chloride and the tetrahydrofuran was distilled off. The residue was recrystallized to give 28.1 g (yield 95%) of 2-butoxyethyl p-formylbenzoate dimethyl acetal.

### Example 1

A 1-ℓ four-necked flask was charged with 59.2 g (0.2 mole) of 2-butoxyethyl p-formylbenzoate dimethyl acetal prepared as in Production Example 1, 18.2 g (0.1 mole) of sorbitol, 200 ml of cyclohexane, 80 ml of methanol and 1.2 g of p-toluenesulfonic acid and the mixture was refluxed with stirring in a nitrogen gas atmosphere for 4.0 hours. Using a Dean-Stark trap, the methanol and byproduct water were removed as desired. Then, after cooling to room temperature, the reaction mixture was neutralized with an aqueous solution of sodium hydroxide and filtered. The white solid thus obtained was washed with warm water and methanol and dried under reduced pressure to recover 55.9 g (yield 86.5%) of the desired 1,3:2,4- bis[p-(2-butoxyethoxycarbonyl)benzylidene]sorbitol as white powder.

### Example 2

(1) Using 21 g (0.1 mole) of methyl p-formylbenzoate dimethyl acetal and 23.1 g (0.14 mole) of 3,6,9-trioxadecyl alcohol (methoxytriglycol), the procedure of Production Example 1 was otherwise followed to give 30.6 g (yield 89.5%) of 3,6,9-trioxadecyl p-formylbenzoate dimethyl acetal.
(2) Using 68.4 g (0.2 mole) of the dimethyl acetal prepared as above (1), the procedure of Example 1 was otherwise followed to give 62.0 g (yield 84.0%) of 1,3:2,4-bis[p-(3,6,9-trioxadecyloxycarbonyl)benzylidene]-sorbitol as white powder.

### Example 3

(1) Using 21 g (0.1 mole) of methyl p-formylbenzoate dimethyl acetal and 74.2 g (0.14 mole) of C₈H₁₇O(CH₂CH₂O)₁₀H, the procedure of Production Example 1 was otherwise followed to give 65.5 g (yield 92.5%) of the corresponding p-formylbenzoate dimethyl acetal.
(2) Using 141.6 g (0.2 mole) of the dimethyl acetal prepared as above, the procedure of Example 1 was otherwise followed to give 122 g (yield 83%) of the contemplated diacetal, viz. 1,3:2,4-bis[p-(octyloxypolyethyleneoxycarbonyl)benzylidene]sorbitol (degree of polymerization of ethylene oxide = 10), as white powder.

### Example 4

(1) Using 21 g (0.1 mole) of methyl p-formylbenzoate dimethyl acetal and 14.3 g (0.14 mole) of n-hexyl alcohol, the procedure of Production Example 1 was otherwise followed to give 26.6 g (yield 95%) of n-hexyl p-formylbenzoate dimethyl acetal.
(2) Using 57.6 g (0.2 mole) of an equimolar mixture of the above dimethyl acetal and 2-butoxyethyl p-formylbenzoate dimethyl acetal, the procedure of Example 3 was otherwise followed to give 55 g (yield 87%) of an approximately equimolar mixture of four different diacetals of general formula (I) wherein A and B are either the same or different and each represents -COOR¹ or -COOR², wherein
   - R¹ = R² = -CH₂CH₂OC₄H₉,
   - R¹ = R² = n-hexyl
   - R¹ = -CH₂CH₂OC₄H₉; R² = n-hexyl
   - R¹ = n-hexyl; R² = -CH₂CH₂OC₄H₉.

### Example 5

(1) Using 21 g (0.1 mole) of methyl p-formylbenzoate dimethyl acetal and 31.1 g (0.5 mole) of ethylene glycol, the procedure of Production Example 1 was otherwise followed to give 22.3 g (yield 93%) of 2-hydroxyethyl p-formylbenzoate dimethyl acetal.
(2) Using 48.0 g (0.2 mole) of the dimethyl acetal prepared as above, the procedure of Example 1 was otherwise followed to give 48.9 g (yield 91.5%) of 1,3:2,4-bis[p-(2-hydroxyethoxycarbonyl)benzylidene]-sorbitol (molecular weight 534).

### Example 6

Using 47.8 g (0.2 mole) of p-(N-2-hydroxyethylcarbamoyl) benzaldehyde dimethyl acetal, the procedure of Example 1 was otherwise followed to give 48.7 g (yield 91.5%) of 1,3:2,4-bis[p-(N-2-hydroxyethylcarbamoyl)benzylidene]sorbitol.

### Example 7

Except that 56.6 g (0.2 mole) of p-(N,N-di-2-hydroxyethylcarbamoyl)benzaldehyde dimethyl acetal was used in lieu of p-(N-2-hydroxyethylcarbamoyl)-benzaldehyde dimethyl acetal, the procedure of Example 1 was repeated to give 55.2 g (yield 89%) of 1,3:2,4-bis[p-(N,N-di-2-hydroxyethylcarbamoyl)benzylidene]sorbitol.

### Example 8

Except that 73 g (0.2 mole) of p-(N-n-decyl-N-2-hydroxyethylcarbamoyl)benzaldehyde dimethyl acetal was used in lieu of p-(N-2-hydroxyethylcarbamoyl)benzaldehyde dimethyl acetal, the procedure of Example 1 was repeated to give 67.1 g (yield 86%) of 1,3:2,4-bis[p-(N-n-decyl-N-2-hydroxyethylcarbamoyl)benzylidene]sorbitol.

### Example 9

Using 28.3 g (0.1 mole) of p-(N,N-di-2-hydroxyethylcarbamoyl)benzaldehyde dimethyl acetal in lieu of 0.2 mole of p-(N-2-hydroxyethylcarbamoyl)benzaldehyde dimethyl acetal, the reaction procedure of Example 1 was repeated at 40°C. Then, 23.9 g (0.1 mole) of p-(N-2-hydroxyethylcarbamoyl)benzaldehyde dimethyl acetal was added and similarly the mixture was stirred for 4 hours. The above procedure gave 49.4 g (yield 85.8%) of 1,3-[p-(N-2-hydroxyethylcarbamoyl)benzylidene]-2,4-[p-(N,N-di-2-hydroxyethylcarbamoyl)benzylidene]sorbitol.

### Example 10

### Production of 1,3:2,4-bis[p-(2-hydroxyethoxycarbonyl)benzylidene]sorbitol

A 1-ℓ flask equipped with a cooling-condenser, thermometer, gas feed piping and stirrer was charged with 47.4 g (0.1 mole) of 1,3:2,4-bis(p-methoxycarbonylbenzylidene)sorbitol, 100 ml of dimethyl sulfoxide, 18.7 g (0.3 mole) of ethylene glycol and 1.08 g (0.02 mole) of sodium methoxide, and the mixture was stirred at 80°C for 3 hours. After cooling to room temperature, the reaction mixture was neutralized with a saturated solution of ammonium chloride and diluted with 500 ml of water. The resulting white solid precipitate was washed with ethanol and dried to give 49.4 g (yield 92.5%) of 1,3:2,4-bis[p-(2-hydroxyethoxycarbonyl)benzylidene]sorbitol.

### Example 11

### Production of 1,3:2,4-bis[p-(2-ethoxyethoxycarbonyl)benzylidene]sorbitol

Using 27 g (0.3 mole) of 2-ethoxyethanol in lieu of ethylene glycol, 100 ml of dimethyl sulfoxide and 1.08 g (0.02 mole) of sodium methoxide, the procedure of Example 10 was followed to give 51.7 g (yield 87.6%) of 1,3:2,4-bis[p-(2-ethoxyethoxycarbonyl)benzylidene]-sorbitol.

### Example 12

### Production of 1,3:2,4-bis[p-(N-2-hydroxyethylcarbamoyl)benzylidene]sorbitol

Using 18.3 g (0.3 mole) of monoethanolamine in lieu of ethylene glycol, 100 ml of dimethyl sulfoxide and 13.5 g (0.25 mole) of sodium methoxide, the procedure of Example 10 was followed to give 50.3 g (yield 94.6%) of 1,3:2,4-bis[p-(N-2-hydroxyethylcarbamoyl)benzylidene]-sorbitol.

### Example 13

### Production of a diacetal compound by ester interchange between 1,3:2,4-bis(p-methoxycarbonylbenzylidene)sorbitol and polyethylene glycol #1000

Using 23.7 g (0.05 mole) of 1,3:2,4-bis(p-methoxycarbonylbenzylidene)sorbitol, 100 ml of dimethyl sulfoxide, 150 g of polyethylene glycol #1000 and 1.08 g (0.02 mole) of sodium methoxide, the procedure of Example 10 was repeated to give 110 g of a diacetal compound having polyethylene glycol #1000 residues as ester residues (yield 90.2%, on an average molecular weight basis).

### Example 14

### Production of 1,3-(p-methylbenzylidene)-2,4-[p-(2-hydroxyethoxycarbonyl)benzylidene]sorbitol

The procedure of Example 1 was repeated except that 2-hydroxyethyl p-formylbenzoate dimethyl acetal was used in an amount of 24 g (0.1 mole) and the reaction was conducted at 40°C for 2 hours. Then, 12 g (0.1 mole) of p-tolualdehyde was added and the mixture was refluxed with stirring for 3 hours to give 39.5 g (yield 85.9%) of 1,3-(p-methylbenzylidene)-2,4-[p-(2-hydroxyethoxycarbonyl)benzylidene]sorbitol.

### Example 15

### Production of 1,3-[p-(2-butoxyethoxycarbonyl) benzylidene]-2,4-(p-methylbenzylidene)sorbitol

Using 43 g (0.1 mole) of 1,3-(p-methoxycarbonylbenzylidene)-2,4-(p-methylbenzylidene)sorbitol as prepared in Example 14, 17.7 g (0.15 mole) of 2-butoxyethanol and 1.08 g (0.02 mole) of sodium methoxide, the procedure of Example 10 was otherwise followed to give 47.3 g (yield 91.7%) of 1,3-[p-(2-butoxyethoxycarbonyl)-benzylidene]-2,4-(p-methylbenzylidene)sorbitol.

### Comparative Example 1

Except that 30 g (0.2 mole) of p-formylbenzoic acid was used in lieu of 2-butoxyethyl p-formylbenzoate, the procedure of Example 1 was repeated to give 26.6 g (yield 59.6%) of 1,3:2,4-bis(carboxybenzylidene)sorbitol.

Then, a 500 ml four-necked flask was charged with 11.2 g (0.25 mole) of 1,3:2,4-bis(carboxybenzylidene)sorbitol prepared above, 100 ml of 2-butoxyethanol and 5 ml of concentrated sulfuric acid and the mixture was refluxed with stirring for about 20 hours. After cooling to room temperature, the resulting crystals were collected by filtration, washed with warm water and methanol, and dried under reduced pressure. The procedure gave 4.4 g (yield 27.2%) of 1,3:2,4-bis[p-(2-butoxyethoxycarbonyl)benzylidene]sorbitol.

## Claims

1. A process for producing a compound of general formula (I) 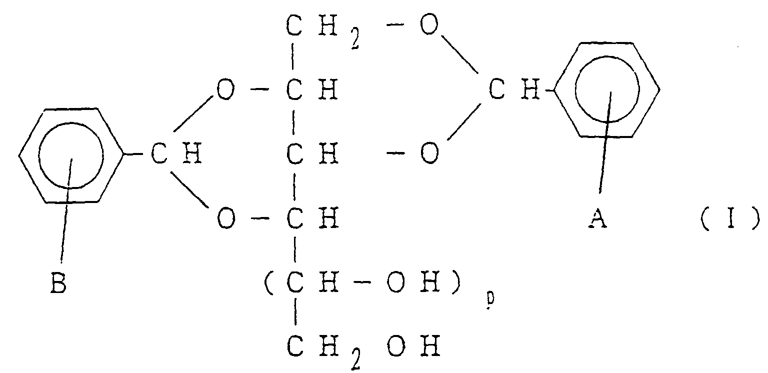 [wherein A and B are the same or different and each represents a hydrogen atom, -(Alk)ₜ (where Alk represents a C₁-C₄ alkyl group and t is equal to 1 through 3), -COOR¹ or -COOR²; or A and B are the same or different and each represents a hydrogen atom, -(Alk)ₜ (wherein Alk represents a C₁-C₄ alkyl group and t is equal to 1 through 3), -CONR³R^{3a} or -CONR⁴R^{4a},
wherein R¹ and R² are the same or different and each represents a C₁-C₄₀ alkyl group, a C₅-C₁₀ cycloalkyl group, a halogen-substituted C₁-C₃₀ alkyl group, a C₃-C₄₀ alkenyl group, a C₃-C₄₀ alkynyl group, a C₄-C₄₀ alkadienyl group, a C₆-C₂₄ alkapolyenyl group having 3 to 6 double bonds, a phenyl or naphthyl group, a C₇-C₄₀ aralkyl group or a residue formed by removing one hydroxyl group from a monohydric or dihydric alcohol selected from the class consisting of polyoxy C₂-C₃ alkylene C₁-C₄₀ alkyl (or C₂-C₄₀ alkenyl) ethers, polyoxy C₂-C₃ alkylene C₁-C₄₀ alkylphenyl ethers, polyoxy(C₂-C₃-alkylene) C₁-C₂₀ alkyl esters, polyoxy (C₂-C₃ alkylene) C₁-C₃₀ alkylamides, polyoxy (C₂-C₃ alkylene) benzoic acid esters, polyesters (Mn=200-100000), polyethylene oxide adducts of primary or secondary amines, rosin alcohol, alkanediols containing 2 to 500 carbon atoms, polyethylene glycols (degree of polymerization = 2-800), polypropylene glycols (degree of polymerization = 2-800), ethylene oxide-propylene oxide copolymers (degree of polymerization = 2-800), 2,2-bis(4-hydroxyphenyl)propane and bis(4-hydroxyphenyl)methane;
R³ and R⁴ are the same or different and each represents a C₁-C₄₀ alkyl group, a C₅-C₁₀ cycloalkyl group, a halogen-substituted C₁-C₃₀ alkyl group, a C₃-C₄₀ alkenyl group, a C₃-C₄₀ alkynyl group, a C₄-C₄₀ alkadienyl group, a C₆-C₂₄ alkapolyenyl group containing 3 to 6 double bonds, a phenyl or naphthyl group or a C₇-C₄₀ aralkyl group; R^{3a} and R^{4a} are the same or different and each represents a hydrogen atom or a group which is the same as R³ or R⁴, or
-NR³R^{3a} and -NR⁴R^{4a} are the same or different and each represents a residue formed by removing one hydrogen atom attached to one nitrogen atom of a primary or secondary mono- or diamine selected from the class consisting of N-(2-hydroxyethyl)-N-(C₁-C₃₀alkyl)amines, mono- or di-C₂-C₃ alkanolamines, alkyldiamines containing 2 to 20 carbon atoms, aryldiamines containing 6 to 20 carbon atoms, aralkyldiamines containing 7 to 20 carbon atoms, and polyoxyethylenediamines (degree of polymerization of ethylene oxide = 1 - 100), polyoxypropylenediamines (degree of polymerization of propylene oxide = 1 - 100) and polyoxyethylene-polyoxypropylenediamine (degree of polymerization = 1 - 100 for each of ethylene oxide and propylene oxide), which can be represented by the general formula
H₂N(T¹O)ₙ(T²O)ₛ-T³-NH₂
[wherein T¹ and T² are different and each represents an ethylene group (-CH₂CH₂-) or a propylene group (-CH₂CH(CH₃)-); T³ is the same as T¹ or T²; n and s are the same or different and each is equal to 0 through 100];
p is equal to 0 or 1;
with the proviso that both of A and B cannot be concurrently hydrogen atom or -(Alk)ₜ but at least one of A and B represents either -COOR¹ or -COOR² or either -CONR³R^{3a} or -CONR⁴R^{4a} and that at least one of R¹ and R² or at least one of R³, R^{3a}, R⁴ and R^{4a} should have at least one group selected from hydroxy group, amino group, ether group, ester group, amide group, halogen atom and rosin alcohol residue],
the process comprising subjecting at least one ester of general formula (IIIa) 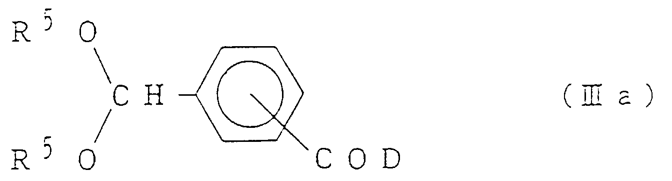 [wherein D represents -OR¹ or -OR² (R¹ and R² are as defined hereinbefore); R⁵ represents an alkyl group of 1 to 4 carbon atoms] or at least one amide of general formula (IIIb) 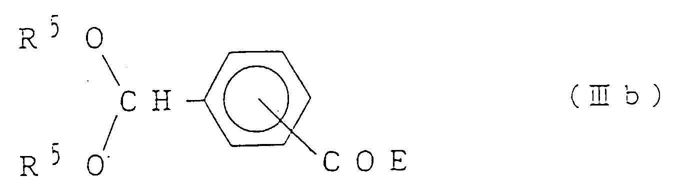 [wherein E represents -NR³R^{3a} or -NR⁴R^{4a} (R³, R^{3a}, R⁴ and R^{4a} are as defined hereinbefore); R⁵ is the same as above]
or (a) an ester of general formula (IIIa) or an amide of general formula (IIIb) and (b) benzaldehyde or a di-lower alkyl acetal thereof or benzaldehyde substituted by 1 to 3 lower alkyl groups or a di-lower alkyl acetal thereof, either in an optional order or as a mixture,
to condensation reaction with a polyhydric alcohol of general formula (II) 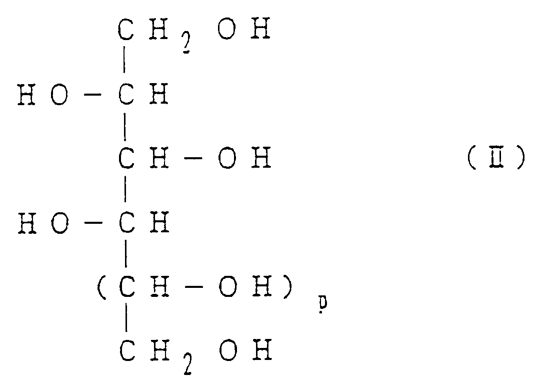 [wherein p is equal to 0 or 1] in a hydrophobic organic solvent and a water-soluble organic solvent, where necessary in the presence of an acid catalyst.

2. A process according to claim 1 wherein A and B are the same and each represents -COOR¹, where R¹ contains at least one member of an ether group, a glycol ether group and a hydroxyl group.

3. A process according to claim 1 wherein A and B are the same and each represents -COOR¹, where R¹ is a residue formed by removing one hydroxyl group from an alcohol selected from the class consisting of alkanediols containing about 2 to 500 carbon atoms poly(C₂-C₃)-alkylene glycols, polyoxyalkylene alkyl or alkenyl ethers of the general formula
R'O(TO)ₙH
[wherein R' represents an alkyl group of 1 to 40 carbon atoms or an alkenyl group of 2 to 40 carbon atoms; T represents an alkylene group of 2 to 3 carbon atoms; n is equal to 1 through 100], polyoxyalkylene alkylphenyl ethers of the general formula 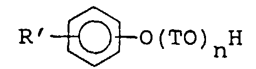 [wherein R' represents an alkyl group of 1 to 40 carbon atoms; T represents an alkylene group of 2 to 3 carbon atoms; n is equal to 1 through 100], N,N-bis[polyoxyalkylene]alkylamides of the general formula
R^{a}CON[(TO)ₘH]₂
[wherein R^{a} represents an alkyl group of 1 to 30 carbon atoms; T represents an alkylene group of 2 to 3 carbon atoms; m is equal to 1 through 80] and rosin alcohols.

4. A process according to claim 1 wherein A and B are the same group and each represents -CONR³R^{3a}, where -NR³R^{3a} is a residue formed by removing one hydrogen atom attached to one nitrogen atom of a primary or secondary mono- or diamine containing at least one member of an ether group, a glycol ether group and a hydroxy group.

5. A process according to claim 1 wherein A and B are the same group and each represents -CONR³R^{3a}, where -NR³R^{3a} is a residue formed by removing one hydrogen atom attached to one nitrogen atom of a primary or secondary mono- or diamine selected from the class consisting of N-(2-hydroxyethyl)-N-(C₁-C₃₀alkyl)amines, mono- or di-C₂-C₃ alkanolamines, alkyldiamines containing 2 to 20 carbon atoms, aryldiamines containing 6 to 20 carbon atoms, aralkyldiamines containing 7 to 20 carbon atoms, and polyoxyethylenediamines (degree of polymerization of ethylene oxide = 1 - 100), polyoxypropylenediamines (degree of polymerization of propylene oxide = 1 - 100) and polyoxyethylene-polyoxypropylenediamines (degree of polymerization = 1 - 100 for each of ethylene oxide and propylene oxide), which is represented by the general formula
H₂N(T¹O)ₙ(T²O)ₛ-T³-NH₂
[wherein T¹ and T² are different and each represents an ethylene group (-CH₂CH₂-) or a propylene group (-CH₂CH(CH₃)-); T³ is the same as T¹ or T²; n and s are the same or different and each is equal to 0 through 100].

6. A process according to claim 1 wherein A and B are the same and each represents -CONR³R^{3a}, where -NR³R^{3a} is a residue formed by removing one hydrogen atom attached to the nitrogen atom of an N-(2-hydroxyethyl)-N-(C₁-C₃₀alkyl)amine or a mono- or di-C₂-C₃ alkanolamine.

7. A process according to claim 1 wherein one of A and B represents a hydrogen atom or -(Alk)ₜ (where Alk and t are as defined in claim 1) with the other being -COOR¹ or -CONR³R^{3a} (where R¹, R³ and R^{3a} are as defined in claim 1).

8. A process for producing a compound of general formula (I) according to claim 1, which comprises subjecting a dialkyl acetal compound of general formula (IV) 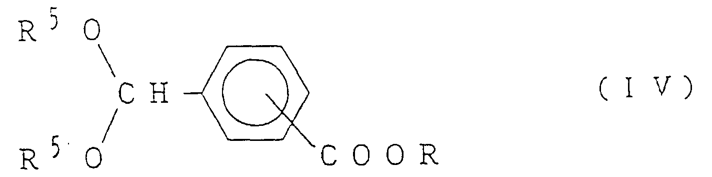 [wherein R⁵ and R each represents an alkyl group of 1 to 4 carbon atoms] to ester interchange or ester-amide interchange reaction with at least one alcohol of the general formulas
R¹OH and R²OH
[wherein R¹ and R² are as defined in claim 1; provided that R¹ and R² are different from R] or at least one amine of the general formulas
R³R^{3a}NH and R⁴R^{4a}NH
[wherein R³, R^{3a}, R⁴ and R^{4a} are as defined in claim 1], and, then, subjecting at least one species of the resulting ester of general formula (IIIa) 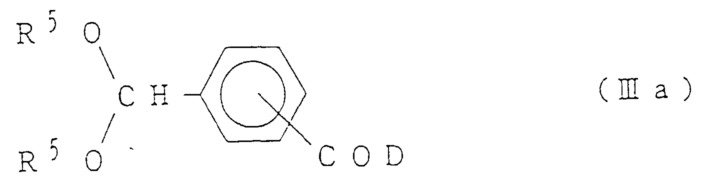 [wherein D represents -OR¹ or -OR² (R¹ and R² are as defined above); R⁵ is as defined above] or at least one species of the resulting amide of general formula (IIIb) 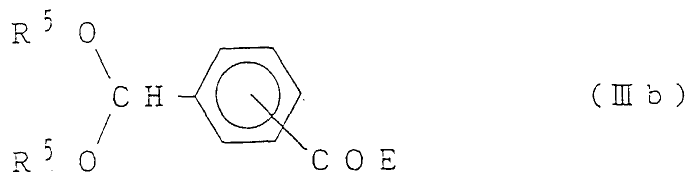 [wherein E represents -NR³R^{3a} or -NR⁴R^{4a} (R³, R^{3a}, R⁴ and R^{4a} are as defined above) ; R⁵ is as defined above], or (a) an ester of general formula (IIIa) or an amide of general formula (IIIb) and (b) benzaldehyde or a di-C₁-C₄ alkyl acetal thereof or benzaldehyde substituted by 1 to 3 C₁-C₄ alkyl groups or a di-C₁-C₄ alkyl acetal thereof, either in an optional order or as a mixture, to condensation reaction with a polyhydric alcohol of general formula (II) 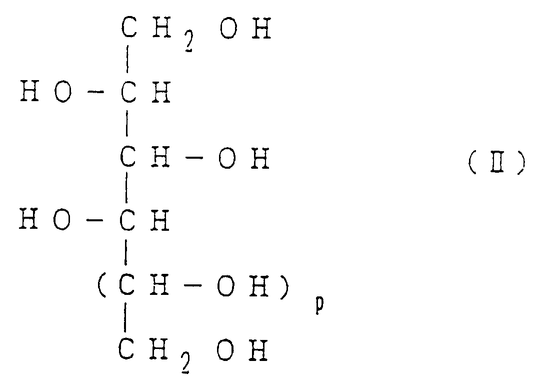 [wherein p is equal to 0 or 1] in a hydrophobic organic solvent and a water-soluble organic solvent, where necessary in the presence of an acid catalyst.

9. A process according to claim 8 wherein the alcohol is an alkanediol of 2 to 500 carbon atoms, a poly(C₂-C₃)alkylene glycol, a poly(degree of polymerization = 1 - 100)oxyalkylene alkyl(C₁-C₄₀) or alkenyl(C₂-C₄₀) ether, a poly(degree of polymerization = 1 - 100)oxyalkylene alkyl(C₁-C₄₀)phenyl ether, or an N,N-bis[poly(degree of polymerization = 1 - 80)-oxyalkylene]alkyl(C₁-C₃₀)amide.

10. A process according to claim 8 wherein the amine is an N-(2-hydroxyethyl)-N-C₁-C₃₀alkylamine, a mono- or di(C₂-C₃)alkanolamine, an alkyldiamine of 2 to 20 carbon atoms, an aryldiamine of 6 to 20 carbon atoms, an aralkyldiamine of 7 to 20 carbon atoms, a polyoxyethylenediamine (degree of polymerization = 1 - 100), a polyoxypropylenediamine (degree of polymerization = 1 - 100) or a polyoxyethylene-polyoxypropylenediamine (degree of polymerization = 1 - 100 for each of ethylene oxide and propylene oxide).

11. A process according to claim 8 wherein the amine is an N-(2-hydroxyethyl)-N-C₁-C₃₀alkylamine or a mono- or di-(C₂-C₃)alkanolamine.

12. A process for producing a compound of general formula (I) according to claim 1, which comprises subjecting a benzaldehyde compound of general formula (V) 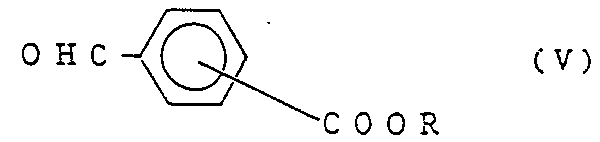 or a di-C₁-C₄ alkyl acetal of general formula (IV) 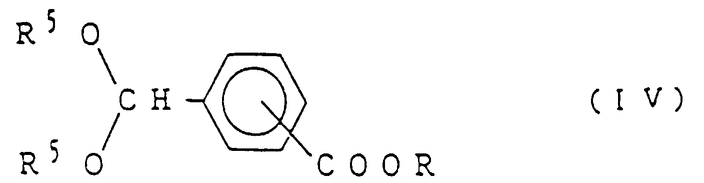 [wherein R⁵ and R each represents an alkyl group of 1 to 4 carbon atoms]
or (a) a compound of general formula (V) or a compound of general formula (IV) and (b) benzaldehyde or a di-C₁-C₄ alkyl acetal thereof or benzaldehyde substituted by 1 to 3 C₁-C₄ alkyl groups or a di-C₁-C₄ alkyl acetal thereof, either in an optional order or as a mixture, to condensation reaction with a polyhydric alcohol of general formula (II) 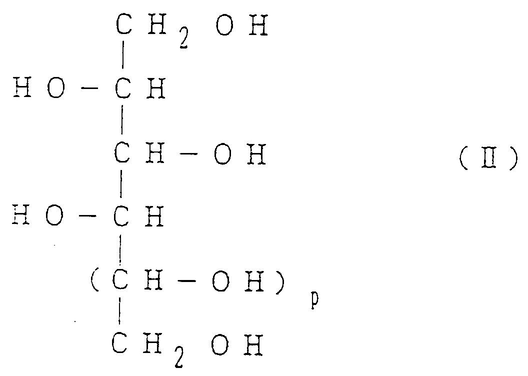 [wherein p is equal to 0 or 1] in a hydrophobic organic solvent and a water-soluble organic solvent, where necessary in the presence of an acid catalyst, and then subjecting the resulting compound of general formula (Ia) 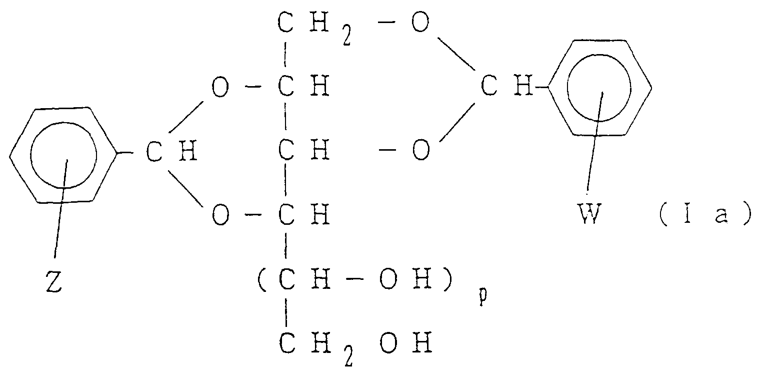 [wherein W and Z are the same or different and each represents a hydrogen atom, -(Alk)ₜ (Alk represents a C₁-C₄ alkyl group; t is equal to 1 through 3) or -COOR (R is as defined above); with the proviso that W and Z cannot concurrently be hydrogen or -(Alk)ₜ; p is equal to 0 or 1] to ester interchange or ester-amide interchange reaction with at least one alcohol of the general formulas
R¹OH and R²OH
[wherein R¹ and R² are as defined in claim 1; with the proviso that R¹ and R² are different from R] or at least one amine of the general formulas
R³R^{3a}NH and R⁴R^{4a}NH
[wherein R³, R^{3a}, R⁴ and R^{4a} are as defined in claim 1].

13. A process for producing a compound of general formula (I) as claimed in claim 1, characterized by subjecting a compound of general formula (Ia) 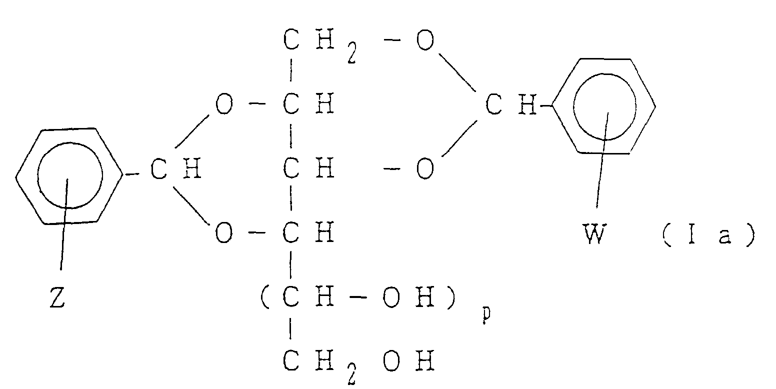 [wherein W and Z are the same or different and each represents a hydrogen atom, -(Alk)ₜ (Alk represents a C₁-C₄ alkyl group; t is equal to 1 through 3) or -COOR (R is as defined above); with the proviso that W and Z cannot concurrently be hydrogen or -(Alk)ₜ; p is equal to 0 or 1] to ester interchange or ester-amide interchange reaction with at least one alcohol of the general formulas
R¹OH and R²OH
[wherein R¹ and R² are as defined in claim 1; with the proviso that R¹ and R² are different from R] or at least one amine of the general formulas
R³R^{3a}NH and R⁴R^{4a}NH
[wherein R³, R^{3a}, R⁴ and R^{4a} are as defined in claim 1].

14. A process according to claim 13 wherein the alcohol is an alkanediol of 2 to 500 carbon atoms, a poly(C₂-C₃)alkylene glycol, a poly(degree of polymerization = 1 - 100)oxyalkylene alkyl(C₁-C₄₀) or alkenyl(C₂-C₄₀) ether, a poly(degree of polymerization = 1 - 100)oxyalkylene alkyl(C₁-C₄₀)phenyl ether, or an N,N-bis[poly(degree of polymerization = 1 - 80)-oxyalkylene]alkyl(C₁-C₃₀)amide.

15. A process according to claim 13 wherein the amine is an N-(2-hydroxyethyl)-N-C₁-C₃₀ alkylamine, a mono- or di(C₂-C₃)alkanolamine, an alkyldiamine of 2 to 20 carbon atoms, an aryldiamine of 6 to 20 carbon atoms, an aralkyldiamine of 7 to 20 carbon atoms, a polyoxyethylenediamine (degree of polymerization = 1 - 100), a polyoxypropylenediamine (degree of polymerization = 1 - 100) or a polyoxyethylene-polyoxypropylene-diamine (degree of polymerization = 1 - 100 for each of ethylene oxide and propylene oxide).

16. A process according to claim 13 wherein the amine is an N-(2-hydroxyethyl)-N-C₁-C₃₀alkylamine or a mono- or di-(C₂-C₃)alkanolamine.

## Patentansprüche

1. Verfahren zur Herstellung einer Verbindung der allgemeinen Formel (I) 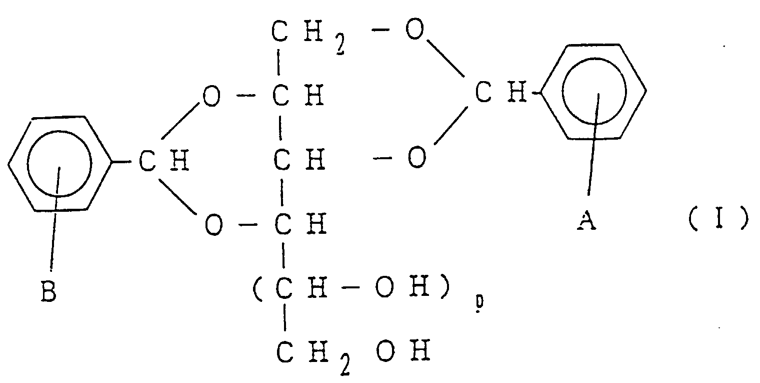 [in der A und B gleich oder verschieden sind und jeweils ein Wasserstoffatom, -(Alk)ₜ (wobei Alk einen C₁-C₄-Alkylrest darstellt und t gleich 1 bis 3 ist), -COOR¹ oder -COOR² darstellen; oder A und B gleich oder verschieden sind und jeweils ein Wasserstoffatom, -(Alk)ₜ (wobei Alk einen C₁-C₄-Alkylrest darstellt und t gleich 1 bis 3 ist), -CONR³R^{3a} oder -CONR⁴R^{4a} darstellen;
wobei R¹ und R² gleich oder verschieden sind und jeweils einen C₁-C₄₀-Alkyl-, C₅-C₁₀-Cycloalkyl-, halogensubstituierten C₁-C₃₀-Alkylrest, einen C₃-C₄₀-Alkenyl-, C₃-C₄₀-Alkinyl-, C₄-C₄₀-Alkadienyl-, C₆-C₂₄-Alkapolyenylrest mit 3 bis 6 Doppelbindungen, eine Phenyl- oder Naphthylgruppe, einen C₇-C₄₀-Aralkylrest oder einen Rest, der durch Entfernen einer Hydroxylgruppe von einem einwertigen oder zweiwertigen Alkohol gebildet wird, ausgewählt aus Polyoxy-C₂-C₃-alkylen-C₁-C₄₀-alkyl- (oder -C₂-C₄₀-alkenyl)ether, Polyoxy-(C₂-C₃-alkylen)-C₁-C₄₀-alkylphenylether, Polyoxy(C₂-C₃-alkylen)-C₁-C₂₀-alkylester, Polyoxy(C₂-C₃)-alkylen-C₁-C₃₀-alkylamiden, Polyoxy(C₂-C₃-alkylen)benzoesäureestern, Polyestern (Mn = 200 - 100000), Polyethylenoxidaddukten von primären oder sekundären Aminen, Harzalkohol, Alkandiolen, die 2 bis 500 Kohlenstoffatome enthalten, Polyethylenglycolen (Polymerisationsgrad = 2 - 800), Polypropylenglycolen (Polymerisationsgrad = 2 - 800), Ethylenoxid-Propylenoxid-Copolymeren (Polymerisationsgrad = 2 - 800), 2,2-Bis(4-hydroxyphenyl)-propan und Bis(4-hydroxyphenyl)methan, darstellt;
R³ und R⁴ gleich oder verschieden sind und jeweils einen C₁-C₄₀-Alkyl-, C₅-C₁₀-Cycloalkyl-, halogensubstituierten C₁-C₃₀-Alkylrest, einen C₃-C₄₀-Alkenyl-, C₃-C₄₀-Alkinyl-, C₄-C₄₀-Alkadienyl-, C₆-C₂₄-Alkapolyenylrest, der 3 bis 6 Doppelbindungen enthält, eine Phenyl- oder Napthylgruppe, oder einen C₇-C₄₀-Aralkylrest darstellt; R^{3a} und R^{4a} gleich oder verschieden sind und jeweils ein Wasserstoffatom oder einen Rest darstellt, der die gleiche Bedeutung wie R³ oder R⁴ aufweist, oder
-NR³R^{3a} und -NR⁴R^{4a} gleich oder verschieden sind und jeweils einen Rest, gebildet durch Entfernen eines Wasserstoffatoms, das an ein Stickstoffatom eines primären oder sekundären Mono- oder Diamins gebunden ist, ausgewählt aus N-(2-Hydroxyethyl)-N-(C₁-C₃₀-alkyl)aminen, Mono- oder Di-C₂-C₃-alkanolaminen, Alkyldiaminen, die 2 bis 20 Kohlenstoffatome enthalten, Aryldiaminen, die 6 bis 20 Kohlenstoffatome enthalten, Aralkyldiaminen, die 7 bis 20 Kohlenstoffatome enthalten, und Polyoxyethylendiaminen (Polymerisationsgrad von Ethylenoxid = 1 - 100), Polyoxypropylendiaminen (Polymerisationsgrad von Propylenoxid = 1 - 100) und Polyoxyethylen-Polyoxypropylen-Diamin (Polymerisationsgrad = 1 - 100 jeweils für Ethylenoxid und Propylenoxid), das durch die allgemeine Formel wiedergegeben werden kann
H₂N(T¹O)ₙ(T²O)ₛ-T³-NH₂
(in der T¹ und T² verschieden sind und jeweils eine Ethylengruppe (-CH₂CH₂-) oder eine Propylengruppe (-CH₂CH(CH₃)-) darstellen; T³ die gleiche Bedeutung wie T¹ oder T² hat; n und s gleich oder verschieden sind und jeweils gleich 0 bis 100 sind), darstellt;
p gleich 0 oder 1 ist;
mit der Maßgabe, daß sowohl A als auch B nicht gleichzeitig ein Wasserstoffatom oder -(Alk)ₜ sein können, aber mindestens einer der Reste A und B entweder -COOR¹ oder -COOR² oder entweder -CONR³R^{3a} oder -CONR⁴R^{4a} darstellt, und daß mindestens einer der Reste R¹ und R² oder mindestens einer der Reste R³, R^{3a}, R⁴ und R^{4a} mindestens einen Rest, ausgewählt aus einer Hydroxylgruppe, Aminogruppe, Ethergruppe, Estergruppe, Amidgruppe, einem Halogenatom und Harzalkoholrest aufweisen sollte],
wobei das Verfahren eine Kondensationsreaktion mindestens eines Esters der allgemeinen Formel (IIIa) 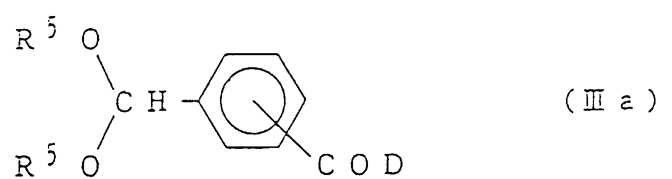 [in der D -OR¹ oder -OR² darstellt (R¹ und R² weisen die vorstehend angegebene Bedeutung auf); R⁵ einen Alkylrest mit 1 bis 4 Kohlenstoffatomen darstellt] oder mindestens eines Amids der allgemeinen Formel (IIIb) 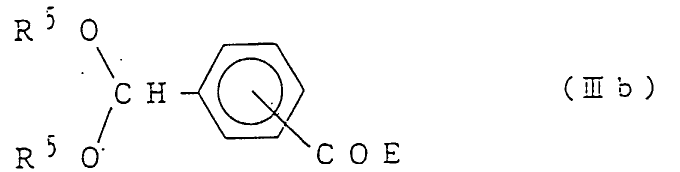 [in der E -NR³R^{3a} oder -NR⁴R^{4a} darstellt (R³, R^{3a}, R⁴ und R^{4a} weisen die vorstehend angegebene Bedeutung auf); R⁵ die vorstehend angegebene Bedeutung aufweist]
oder (a) eines Esters der allgemeinen Formel (IIIa) oder eines Amids der allgemeinen Formel (IIIb) und (b) Benzaldehyd oder eines Diniederalkylacetals davon oder eines mit 1 bis 3 Niederalkylresten substituierten Benzaldehyds oder eines Diniederalkylacetals davon, entweder in beliebiger Reihenfolge oder als Gemisch mit einem mehrwertigen Alkohol der allgemeinen Formel (II) 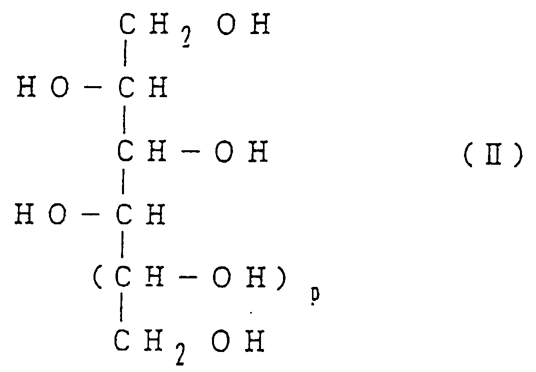 [wobei p gleich 0 oder 1 ist] in einem hydrophoben organischen Lösungsmittel und in einem wasserlöslichen organischen Lösungsmittel, falls erforderlich in Gegenwart eines sauren Katalysators.

2. Verfahren nach Anspruch 1, wobei A und B gleich sind und jeweils -COOR¹ darstellen, wobei R¹ mindestens einen Vertreter einer Ethergruppe, einer Glycolethergruppe und einer Hydroxylgruppe enthält.

3. Verfahren nach Anspruch 1, wobei A und B gleich sind und jeweils -COOR¹ darstellen, wobei R¹ ein Rest, gebildet durch Entfernen einer Hydroxylgruppe von einem Alkohol, ausgewählt aus Alkandiolen, die etwa 2 bis 500 Kohlenstoffatome enthalten, Poly(C₂-C₃)alkylenglycolen, Polyoxyalkylenalkyl- oder -alkenylether der allgemeinen Formel
R'O(TO)ₙH
[wobei R' einen Alkylrest mit 1 bis 40 Kohlenstoffatomen oder einen Alkenylrest mit 2 bis 40 Kohlenstoffatomen darstellt; T einen Alkylenrest mit 2 bis 3 Kohlenstoffatomen darstellt; n gleich 1 bis 100 ist], Polyoxyalkylenalkylphenylethern der allgemeinen Formel 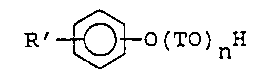 [wobei R' einen Alkylrest mit 1 bis 40 Kohlenstoffatomen darstellt; T einen Alkylenrest mit 2 bis 3 Kohlenstoffatomen darstellt; n gleich 1 bis 100 ist], N,N-Bis[polyoxyalkylen]alkylamiden der allgemeinen Formel
R^{a}CON[(TO)ₘH]₂
[wobei R^{a} einen Alkylrest mit 1 bis 30 Kohlenstoffatomen darstellt; T einen Alkylenrest mit 2 bis 3 Kohlenstoffatomen darstellt; m gleich 1 bis 80 ist] und Harzalkoholen.

4. Verfahren nach Anspruch 1, wobei A und B gleich sind und jeweils -CONR³R^{3a} darstellen, wobei -NR³R^{3a} ein Rest ist, der durch Entfernen eines Wasserstoffatoms gebildet wird, das an ein Stickstoffatom eines primären oder sekundären Mono- oder Diamins gebunden ist, das mindestens einen Vertreter einer Ethergruppe, Glycolethergruppe und Hydroxylgruppe enthält.

5. Verfahren nach Anspruch 1, wobei A und B gleich sind und jeweils -CONR³R^{3a} darstellen, wobei -NR³R^{3a} ein Rest ist, der durch Entfernen eines Wasserstoffatoms gebildet wird, das an ein Stickstoffatom eines primären oder sekundären Mono- oder Diamins gebunden ist, ausgewählt aus N-(2-Hydroxyethyl)-N-(C₁-C₃₀-alkyl)aminen, Mono- oder Di-C₂-C₃-alkanolaminen, Alkyldiaminen mit 2 bis 20 Kohlenstoffatomen, Aryldiaminen mit 6 bis 20 Kohlenstoffatomen, Aralkyldiaminen mit 7 bis 20 Kohlenstoffatomen und Polyoxyethylendiaminen (Polymerisationsgrad von Ethylenoxid = 1 - 100), Polyoxypropylendiaminen (Polymerisationsgrad von Propylenoxid = 1 - 100) und Polyoxyethylen-Polyoxypropylen-Diaminen (Polymerisationsgrad = 1 - 100, jeweils für Ethylenoxid und Propylenoxid), die durch die allgemeine Formel wiedergegeben werden
H₂N(T¹O)ₙ(T²O)ₛ-T³-NH₂
[in der T¹ und T² verschieden sind und jeweils eine Ethylengruppe (-CH₂CH₂-) oder eine Propylengruppe (-CH₂CH(CH₃)-) darstellen; T³ die gleiche Bedeutung wie T¹ oder T² aufweist; n und s gleich oder verschieden sind und jeweils gleich 0 bis 100 sind].

6. Verfahren nach Anspruch 1, wobei A und B gleich sind und jeweils -CONR³R^{3a} darstellen, wobei -NR³R^{3a} ein Rest ist, gebildet durch Entfernen eines Wasserstoffatoms, das an das Stickstoffatom eines N-(2-Hydroxyethyl)-N-(C₁-C₃₀-alkyl)amins oder eines Mono- oder Di-C₂-C₃-alkanolamins gebunden ist.

7. Verfahren nach Anspruch 1, wobei einer der Reste A und B ein Wasserstoffatom oder -(Alk)ₜ darstellt (wobei Alk und t die in Anspruch 1 angegebene Bedeutung aufweisen), wobei der andere Rest -COOR¹ oder -CONR³R^{3a} ist (wobei R¹, R³ und R^{3a} die in Anspruch 1 angegebene Bedeutung haben).

8. Verfahren zur Herstellung einer Verbindung der allgemeinen Formel (I) nach Anspruch 1, umfassend eine Esteraustausch- oder Esteramidaustauschreaktion einer Dialkylacetalverbindung der allgemeinen Formel (IV) 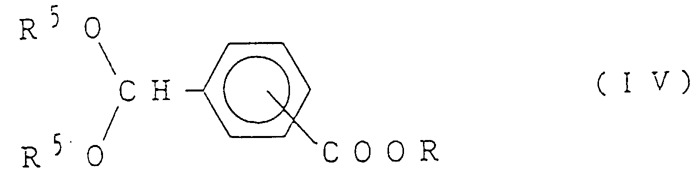 [in der R⁵ und R jeweils einen Alkylrest mit 1 bis 4 Kohlenstoffatomen darstellen] mit mindestens einem Alkohol der allgemeinen Formeln
R¹OH und R²OH
[wobei R¹ und R² die in Anspruch 1 angegebene Bedeutung aufweisen; mit der Maßgabe, daß R¹ und R² von R verschieden sind] oder mindestens einem Amin der allgemeinen Formeln
R³R^{3a}NH und R⁴R^{4a}NH
[wobei R³, R^{3a}, R⁴ und R^{4a} die in Anspruch 1 angegebene Bedeutung aufweisen], und dann Durchführung einer Kondensationsreaktion mindestens einer Art des entstehenden Esters der allgemeinen Formel (IIIa) 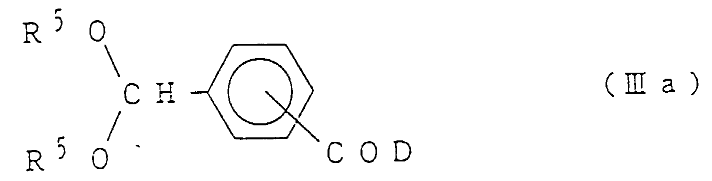 [in der D -OR¹ oder -OR² darstellt (R¹ und R² weisen die vorstehend angegebene Bedeutung auf); R⁵ die vorstehend angegebene Bedeutung aufweist] oder mindestens einer Art des entstehenden Amids der allgemeinen Formel (IIIb) 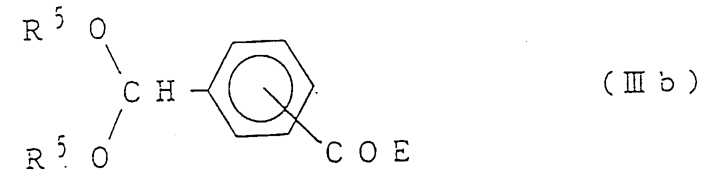 [in der E -NR³R^{3a} oder -NR⁴R^{4a} darstellt (R³, R^{3a}, R⁴ und R^{4a} weisen die vorstehend angegebene Bedeutung auf); R⁵ die vorstehend angegebene Bedeutung aufweist] oder (a) eines Esters der allgemeinen Formel (IIIa) oder eines Amids der allgemeinen Formel (IIIb) und (b) Benzaldehyd oder eines Di-C₁-C₄-alkylacetals davon oder eines mit 1 bis 3 C₁-C₄-Alkylresten substituierten Benzaldehyds oder eines Di-C₁-C₄-alkylacetals davon entweder in beliebiger Reihenfolge oder als Gemisch mit einem mehrwertigen Alkohol der allgemeinen Formel (II) 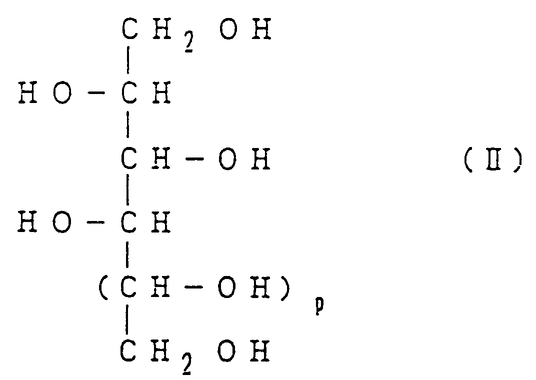 [wobei p gleich 0 oder 1 ist] in einem hydrophoben organischen Lösungsmittel und einem wasserlöslichen organischen Lösungsmittel, falls erforderlich in Gegenwart eines sauren Katalysators.

9. Verfahren nach Anspruch 8, wobei der Alkohol ein Alkandiol mit 2 bis 500 Kohlenstoffatomen, ein Poly(C₂-C₃)alkylenglycol, ein Poly(Polymerisationsgrad = 1 - 100)oxyalkylenalkyl(C₁-C₄₀)- oder -alkenyl(C₂-C₄₀)ether, ein Poly(Polymerisationsgrad = 1 - 100)oxyalkylenalkyl(C₁-C₄₀)phenylether oder ein N,N-Bis[poly(Polymerisationsgrad = 1 - 80)oxyalkylen]alkyl(C₁-C₃₀)amid ist.

10. Verfahren nach Anspruch 8, wobei das Amin ein N-(2-Hydroxyethyl)-N-C₁-C₃₀-alkylamin, ein Mono- oder Di(C₂-C₃)alkanolamin, ein Alkyldiamin mit 2 bis 20 Kohlenstoffatomen, ein Aryldiamin mit 6 bis 20 Kohlenstoffatomen, ein Aralkyldiamin mit 7 bis 20 Kohlenstoffatomen, ein Polyoxyethylendiamin (Polymerisationsgrad = 1 - 100), ein Polyoxypropylendiamin (Polymerisationsgrad = 1 - 100) oder ein Polyoxyethylen-Polyoxypropylendiamin (Polymerisationsgrad = 1 - 100, jeweils für Ethylenoxid und Propylenoxid) ist.

11. Verfahren nach Anspruch 8, wobei das Amin ein N-(2-Hydroxyethyl)-N-C₁-C₃₀-alkylamin oder ein Mono- oder Di(C₂-C₃)alkanolamin ist.

12. Verfahren zur Herstellung einer Verbindung der allgemeinen Formel (I) nach Anspruch 1, umfassend eine Kondensationsreaktion einer Benzaldehydverbindung der allgemeinen Formel (V) 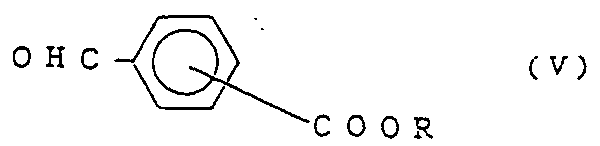 oder eines Di-C₁-C₄-alkylacetals der allgemeinen Formel (IV) 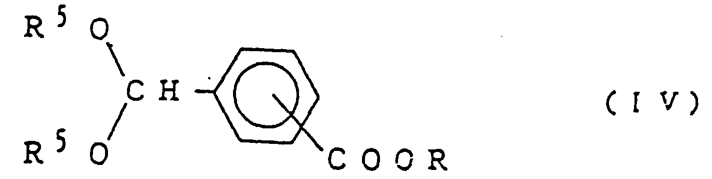 [in der R⁵ und R jeweils einen Alkylrest mit 1 bis 4 Kohlenstoffatomen darstellen]
oder (a) einer Verbindung der allgemeinen Formel (V) oder einer Verbindung der allgemeinen Formel (IV) und (b) Benzaldehyd oder ein Di-C₁-C₄-alkylacetal davon oder eines mit 1 bis 3 C₁-C₄-Alkylresten substituierten Benzaldehyds oder eines Di-C₁-C₄-alkylacetals davon, entweder in beliebiger Reihenfolge oder als Gemisch, mit einem mehrwertigen Alkohol der allgemeinen Formel (II) 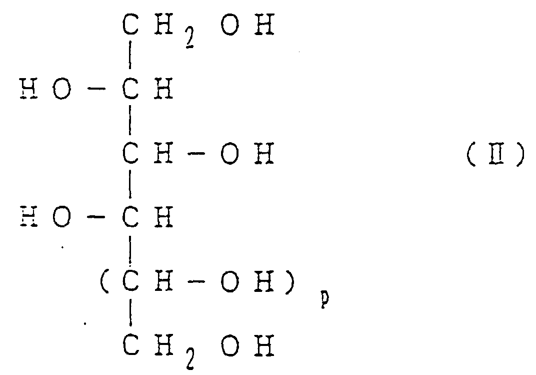 [in der p gleich 0 oder 1 ist] in einem hydrophoben organischen Lösungsmittel und einem wasserlöslichen organischen Lösungsmittel, falls erforderlich in Gegenwart eines sauren Katalysators, und dann eine Esteraustausch- oder Esteramidaustauschreaktion der entstandenen Verbindung der allgemeinen Formel (Ia) 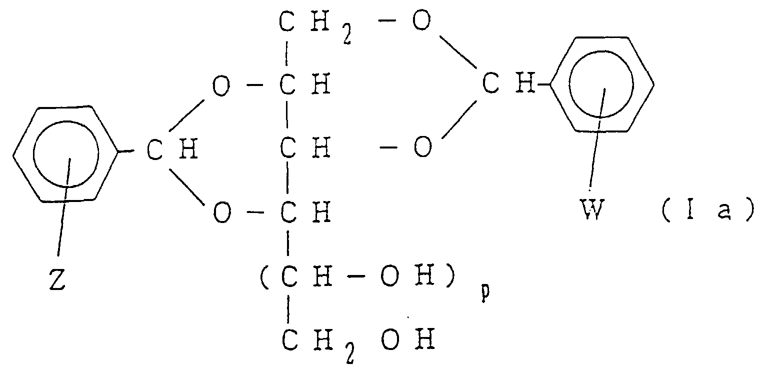 [in der W und Z gleich oder verschieden sind und jeweils ein Wasserstoffatom, -(Alk)ₜ (Alk stellt einen C₁-C₄-Alkylrest dar; t ist gleich 1 bis 3) oder -COOR (R weist die vorstehend angegebene Bedeutung auf) darstellt; mit der Maßgabe, daß W und Z nicht gleichzeitig ein Wasserstoffatom oder -(Alk)ₜ sein können; p gleich 0 oder 1 ist] mit mindestens einem Alkohol der allgemeinen Formeln
R¹OH und R²OH
[wobei R¹ und R² die in Anspruch 1 angegebene Bedeutung haben; mit der Maßgabe, daß R¹ und R² von R verschieden sind] oder mindestens einem Amin der allgemeinen Formeln
R³R^{3a}NH und R⁴R^{4a}NH
[wobei R³, R^{3a}, R⁴ und R^{4a} die in Anspruch 1 angegebene Bedeutung haben].

13. Verfahren zur Herstellung einer Verbindung der allgemeinen Formel (I) nach Anspruch 1, gekennzeichnet durch eine Esteraustausch- oder Esteramidaustauschreaktion einer Verbindung der allgemeinen Formel (Ia) 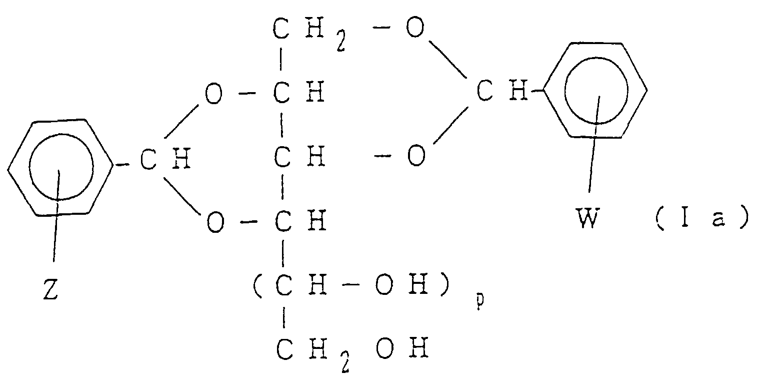 [in der W und Z gleich oder verschieden sind und jeweils ein Wasserstoffatom, -(Alk)ₜ (Alk stellt einen C₁-C₄-Alkylrest dar; t ist gleich 1 bis 3) oder -COOR (R weist die vorstehend angegebene Bedeutung auf) darstellt; mit der Maßgabe, daß W und Z nicht gleichzeitig ein Wasserstoffatom oder -(Alk)ₜ sein können; p gleich 0 oder 1 ist] mit mindestens einem Alkohol der allgemeinen Formeln
R¹OH und R²OH
[wobei R¹ und R² die in Anspruch 1 angegebene Bedeutung haben; mit der Maßgabe, daß R¹ und R² von R verschieden sind] oder mindestens einem Amin der allgemeinen Formeln
R³R^{3a}NH und R⁴R^{4a}NH
[wobei R³, R^{3a}, R⁴ und R^{4a} die in Anspruch 1 angegebene Bedeutung haben].

14. Verfahren nach Anspruch 13, wobei der Alkohol ein Alkandiol mit 2 bis 500 Kohlenstoffatomen, ein Poly(C₂-C₃)alkylenglycol, ein Poly(Polymerisationsgrad = 1 - 100)oxyalkylenalkyl(C₁-C₄₀)- oder -alkenyl(C₂-C₄₀)ether, ein Poly(Polymerisationsgrad = 1 - 100)oxyalkylenalkyl(C₁-C₄₀)phenylether oder ein N,N-Bis[poly(Polymerisationsgrad = 1 - 80)oxyalkylen]alkyl(C₁-C₃₀)amid ist.

15. Verfahren nach Anspruch 13, wobei das Amin ein N-(2-Hydroxyethyl)-N-C₁-C₃₀-alkylamin, ein Mono- oder Di(C2-C3)alkanolamin, ein Alkyldiamin mit 2 bis 20 Kohlenstoffatomen, ein Aryldiamin mit 6 bis 20 Kohlenstoffatomen, ein Aralkyldiamin mit 7 bis 20 Kohlenstoffatomen, ein Polyoxyethylendiamin (Polymerisationsgrad = 1 - 100), ein Polyoxypropylendiamin (Polymerisationsgrad = 1 - 100) oder ein Polyoxyethylen-Polyoxypropylen-Diamin (Polymerisationsgrad = 1 - 100, jeweils für Ethylenoxid und Propylenoxid) ist.

16. Verfahren nach Anspruch 13, wobei das Amin ein N-(2-Hydroxyethyl)-N-C₁-C₃₀-alkylamin oder ein Mono- oder Di(C₂-C₃)alkanolamin ist.

## Revendications

1. Procédé pour la production d'un composé de formule générale (I) : 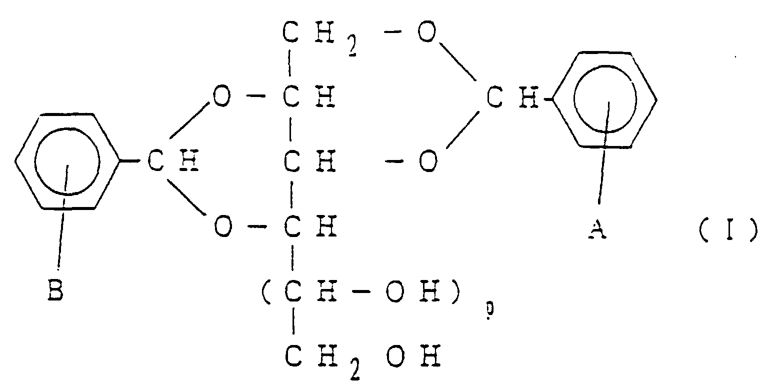 [- dans laquelle A et B sont identiques ou différents, et représentent chacun un atome d'hydrogène, un groupe -(Alk)ₜ (où Alk représente un groupe alkyle C₁-C₄, et t varie de 1 à 3), -COOR¹, ou -COOR²; ou A et B sont identiques ou différents, et représentent chacun un atome d'hydrogène, un groupe -(Alk)ₜ (où Alk représente un groupe alkyle C₁-C₄, et t est égal à 1, 2, ou 3), -CONR³R^{3a}, ou CONR⁴R^{4a};
- dans laquelle R¹ et R² sont identiques ou différents et représentent chacun un groupe alkyle C₁-C₄₀, un groupe cycloalkyle C₅-C₁₀, un groupe alkyle C₁-C₃₀ substitué par un halogène, un groupe alkényle C₃-C₄₀, un groupe alkynyle C₃-C₄₀, un groupe alcadiényle C₄-C₄₀, un groupe alcapolyényle C₆-C₂₄ comportant 3 à 6 doubles liaisons, un groupe phényle ou naphtyle, un groupe aralkyle C₇-C₄₀, ou un résidu formé par l'élimination d'un groupe hydroxyle d'un alcool monohydrique ou dihydrique choisi au sein de l'ensemble constitué par les éthers polyoxyalkylène C₂-C₃ alkyl C₁-C₄₀ (ou alkényle C₂-C₄₀), les éthers polyoxyalkylène C₂-C₃ alkyl C₁-C₄₀ alkylphényle, les esters polyoxy(alkylène C₂-C₃)alkyl C₁-C₂₀, les polyoxy(alkyléne C₂-C₃)alkylamides C₁-C₃₀, les esters polyoxy(alkylène C₂-C₃) d'acide benzoïque, les polyesters (Mn=200 à 100.000), les addititifs d'oyde de polyéthylène d'amines primaires ou secondaires, l'alcool rosinique, les alcanediols contenant de 2 à 500 atomes de carbone, les polyéthylèneglycols (degré de polymérisation = 2 à 800), les polypropylèneglycols (degré de polymérisation = 2 à 800), les copolymères oxyde d'éthylène/oxyde de propylène (degré de polymérisation = 2 à 800), le 2,2-bis(4-hydroxyphényl)propane, et le bis(4-hydroxyphényl)méthane ;
- R³ et R⁴ sont identiques ou différents et représentent chacun un groupe alkyle C₁-C₄₀, un groupe cycloalkyle C₅-C₁₀, un groupe alkyle C₁-C₃₀ substitué par un halogène, un groupe alkényle C₃-C₄₀, un groupe alkynyle C₃-C₄₀, un groupe alcadiényle C₄-C₄₀, un groupe alcapolyényle C₆-C₂₄ comportant 3 à 6 doubles liaisons, un groupe phényle ou naphtyle, ou un groupe aralkyle C₇-C₄₀;
- R^{3a} et R^{4a} sont identiques ou différents et représentent chacun un atome d'hydrogène ou un groupe qui est le même que R³ ou R⁴, ou -NR³R^{3a} et -NR⁴R^{4a} sont identiques ou différents et représentent chacun un résidu formé par l'élimination d'un atome d'hydrogène attaché à un atome d'azote d'une monoamine ou d'une diamine primaire ou secondaire choisie au sein de l'ensemble comprenant les N-(2-hydroxyéthyl)-N-(alkyl C₁-C₃₀)amines, les mono- ou les di-alcanol(C₂-C₃)-amines, les alkyldiamines contenant de 2 à 20 atomes de carbone, les aryldiamines contenant de 6 à 20 atomes de carbone, les aralkyldiamines contenant de 7 à 20 atomes de carbone, et les polyoxyéthylènediamines (degré de polymérisation d'oxyde d'éthylène = 1 à 100), les polyoxypropylènediamines (degré de polymérisation d'oxyde de propylène = 1 à 100), et les polyoxyéthylène/polyoxypropylènediamines (degré de polymérisation pour l'oxyde d'éthylène et pour l'oxyde de propylène = 1 à 100) pouvant être représentés par la formule générale suivante :
H₂N(T¹O)ₙ(T²O)ₛ-T³-NH₂
(dans laquelle T¹ ou T² sont différents et représentent chacun un groupe éthylène (-CH₂-CH₂-) ou un groupe propylène [-CH₂CH(CH₃)-]; T³ est identique à T¹ ou à T² n et s sont identiques ou différents et chacun varie de 0 à 100); p est égal à 0 ou à 1 ; à la condition que A et B ne puissent pas être concurremment un atome d'hydrogène ou un groupe -(Alk)ₜ mais qu'au moins l'un de A ou de B représente soit un groupe -COOR¹ soit un groupe -COOR², ou un des groupes -CONR³R^{3a} ou -CONR⁴R^{4a} et qu'au moins l'un de R¹ ou de R², ou au moins l'un de R³, R^{3a}, R⁴ ou R^{4a} doive avoir au moins un groupe choisi parmi les groupes hydroxy, amino, éther, ester, amide, les atomes d'halogène, et le résidu d'alcool rosinique]
ledit procédé comprenant la soumission d'au moins un ester de formule générale (IIIa) : 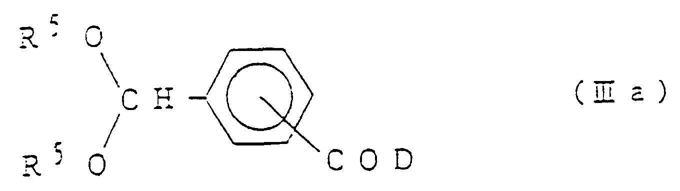 [dans laquelle D représente -OR¹ ou -OR² (R¹ et R² étant tels que définis plus haut); R⁵ représente un groupe alkyle contenant de 1 à 4 atomes de carbone], ou d'au moins un amide de formule générale (IIIb) : 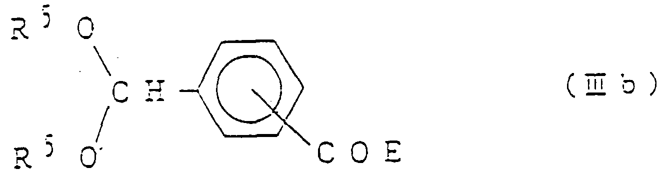 [dans laquelle E représente -NR³R^{3a} ou -NR⁴R^{4a} (R³, R^{3a}, R⁴, et R^{4a} étant tels que définis plus haut) ; R⁵ est le même que ci-dessus],
ou (a) d'un ester de formule générale (IIIa) ou d'un amide de formule générale (IIIb), et (b) du benzaldéhyde ou d'un dialkylacétal inférieur de ce dernier, ou de benzaldéhyde substitué par 1 à 3 groupes alkyle inférieur, ou d'un dialkylacétal inférieur de ce dernier, soit dans un ordre éventuel, soit sous forme de mélange,
à une réaction de condensation avec un alcool polyhydrique de formule générale (II) : 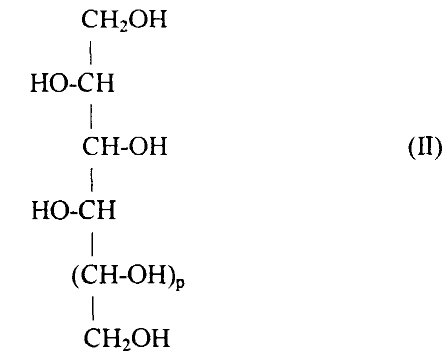 [dans laquelle p est égal à 0 ou à 1] dans un solvant organique hydrophobe et un solvant organique hydrosoluble, si nécessaire en présence d'un catalyseur acide.

2. Procédé selon la revendication 1, dans lequel A et B sont identiques et représentent chacun un groupe -COOR¹, où R¹ contient au moins un représentant choisi parmi les groupes éther, éther de glycol, et hydroxyle.

3. Procédé selon la revendication 1, dans lequel A et B sont identiques et représentent chacun un groupe -COOR¹ où R¹ est un résidu formé par l'élimination d'un groupe hydroxyle de l'alcool choisi au sein de l'ensemble comprenant les alcanediols contenant environ de 2 à 500 atomes de carbone, les polyalkylène(C₂-C₃)glycols, les éthers de polyoxyalkylène alkyle ou alkényle de formule générale:
R'O(TO)ₙH
[dans laquelle R' représente un groupe alkyle contenant de 1 à 40 atomes de carbone ou un groupe alkényle contenant de 2 à 40 atomes de carbone ; T représente un groupe alkylène contenant de 2 à 3 atomes de carbone ; n varie entre 1 et 100], les éthers de polyoxyalkylène alkylphényle de formule générale : 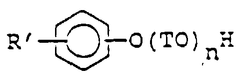 [dans laquelle R' représente un groupe alkyle contenant de 1 à 40 atomes de carbone ; T représente un groupe alkylène contenant de 2 à 3 atomes de carbone ; n varie entre 1 et 100], les N,N-bis(polyoxyalkylène)alkylamides de formule générale :
R^{a}CON[(TO)ₘH]₂
[dans laquelle R^{a} représente un groupe alkyle contenant de 1 à 30 atomes de carbone; T représente un groupe alkylène contenant de 2 à 3 atomes de carbone ; m varie entre 1 et 80], et les alcools rosiniques.

4. Procédé selon la revendication 1, dans lequel A et B représentent le même groupe et chacun représente -CONR³R^{3a} où -NR³R^{3a} est un résidu formé par l'élimination d'un atome d'hydrogène attaché à un atome d'azote d'une mono- ou diamine primaire ou secondaire contenant au moins un membre choisi parmi les groupes éther, éther de glycol, et hydroxy.

5. Procédé selon la revendication 1, dans lequel A et B représentent le même groupe et chacun représente -CONR³R^{3a} où -NR³R^{3a} est un résidu formé par l'élimination d'un atome d'hydrogène attaché à un atome d'azote d'une mono- ou diamine primaire ou secondaire choisie au sein de l'ensemble comprenant les N-(2-hydroxyéthyl)-N-(alkyl C₁-C₃₀)amines, des mono- ou les di-(alcanol C₂-C₃)-amines, les alkyldiamines contenant de 2 à 20 atomes de carbone, les aryldiamines contenant de 6 à 20 atomes de carbone, les aralkyldiamines contenant de 7 à 20 atomes de carbone, et les polyoxyéthylènediamines (degré de polymérisation d'oxyde d'éthylène = 1 à 100), les polyoxypropylènediamines (degré de polymérisation d'oxvde de propylène = 1 à 100), et les polyoxyéthylène/polyoxypropylènediamines (degré de polymérisation pour l'oxyde d'éthylène et pour l'oxyde de propylène = 1 à 100) pouvant être représentée par la formule générale suivante :
H₂N(T¹O)ₙ(T²O)ₛ-T³-NH₂
[dans laquelle T¹ et T² sont différents et représentent chacun un groupe éthylène (-CH₂-CH₂-) ou un groupe propylène (-CH₂CH(CH₃)-); T³ est identique à T¹ et à T² ; n et s sont identiques ou différents et chacun varie de 0 à 100].

6. Procédé selon la revendication 1, dans lequel A et B sont identiques et représentent chacun -CONR³R^{3a} où -NR³R^{3a} est un résidu formé par l'élimination d'un atome d'hydrogène attaché à un atome d'azote d'une N-(2-hydroxyéthyl)-N-(alkyl C₁-C₃₀)amine ou d'une mono- ou di-(alcanol C₂-C₃)amine.

7. Procédé selon la revendication 1, dans lequel l'un de A ou de B représente un atome d'hydrogène ou un groupe -(Alk)ₜ (où Alk et t sont tels que définis dans la revendication 1), l'autre étant -COOR¹ ou -CONR³R^{3a} (où R¹, R³, et R^{3a} sont tels que définis dans la revendication 1).

8. Procédé de production d'un composé de formule générale (I) selon la revendication 1, qui comprend la soumission d'un composé dialkylacétal de formule générale (IV) : 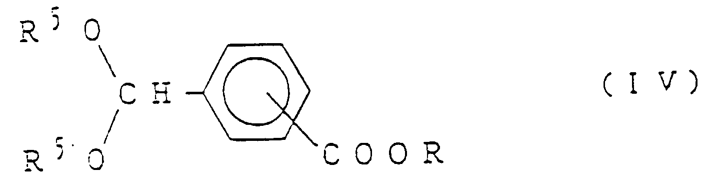 [dans laquelle R⁵ et R représentent chacun un groupe alkyle contenant de 1 à 4 atomes de carbone], à une réaction d'échange inter-ester ou ester-amide avec au moins un alcool de formules générales :
R¹OH et R²OH
[dans lesquelles R¹ et R² sont tels que définis dans la revendication 1 ; à la condition que R¹ et R² soient différents de R], ou avec au moins une amine de formules générales :
R³R^{3a}NH et R⁴R^{4a}NH
[dans lesquelles R³, R^{3a}, R⁴, et R^{4a} sont tels que définis dans la revendication 1], puis la soumission d'au moins une espèce de l'ester ainsi obtenu, de formule générale (IIIa) : 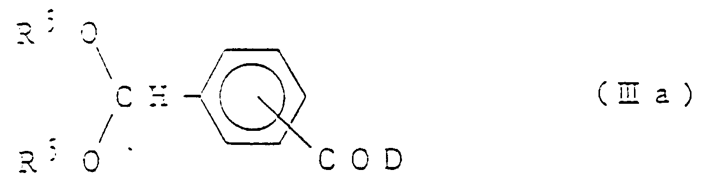 [dans lesquelles D représente -OR¹ ou -OR² (R¹ et R² étant tels que définis plus haut) ; R⁵ est tel que défini plus haut],
ou d'au moins une espèce de l'amide ainsi obtenu, de formule générale (IIIb): 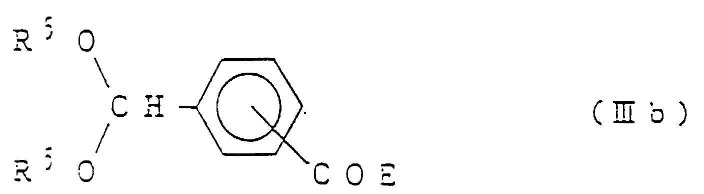 [dans laquelle E représente -NR³R^{3a} ou -NR⁴R^{4a}, (R³, R^{3a}, R⁴, et R^{4a} sont tels que définis plus haut) ; R⁵ est tel que défini plus haut],
ou (a) d'un ester de formule générale (IIIa) ou d'un amide de formule générale (IIIb) et (b) de benzaldéhyde ou de di(alkyl C₁-C₄)acétal de ce dernier, ou de benzaldéhyde substitué par 1 à 3 groupes alkyle C₁-C₄, ou d'un di(alkyl C₁-C₄)acétal de ce dernier, soit dans un ordre éventuel, soit sous forme de mélange, à une réaction de condensation avec un alcool polyhydrique de formule générale (II) : 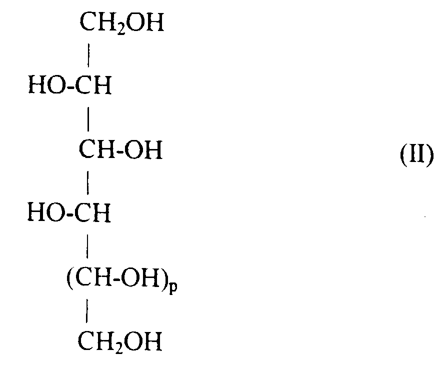 [dans laquelle p est égal à 0 ou à 1] dans un solvant organique hydrophobe et un solvant organique hydrosoluble, si nécessaire en présence d'un catalyseur acide.

9. Procédé selon la revendication 8, dans lequel l'alcool est un alcanediol contenant de 2 à 500 atomes de carbone, un poly(alkylène-C₂-C₃)glycol, un poly(degré de polymérisation = 1 à 100)oxyalkylène alkyl(C₁-C₄₀) ou alkényl(C₂-C₄₀) éther, un poly(degré de polymérisation = 1 à 100)oxyalkylène alkyl(C₁-C₄₀)phényl éther, ou un N,N-bis-[poly(degré de polymérisation = 1 à 80)oxyalkylène]alkyl(C₁-C₃₀)-amide.

10. Procédé selon la revendication 8, dans lequel l'amine est la N-(2-hydroxyéthyl)-N-alkyl(C₁-C₃₀)amine, une mono- ou une dialcanol(C₂-C₃)amine, une alkyldiamine contenant de 2 à 20 atomes de carbone, une aryldiamine contenant de 6 à 20 atomes de carbone, une aralkyldiamine contenant de 7 à 20 atomes de carbone , une polyoxyéthylènediamine (degré de polymérisation = 1 à 100), une polyoxypropylènediamine (degré de polymérisation = 1 à 100), ou une polyoxyéthylène/polyoxypropylènediamine (degré de polymérisation = 1 à 100 pour l'oxyde d'éthylène et pour l'oxyde de propylène).

11. Procédé selon la revendication 8, dans lequel l'amine est une N-(2-hydroxyéthyl)-N-alkyl(C₁-C₃₀)amine, ou une mono- ou dialcanol(C₂-C₃)amine.

12. Procédé de production d'un composé de formule générale (I) selon la revendication 1, qui comprend la soumission d'un composé benzaldéhyde de formule générale (V) : 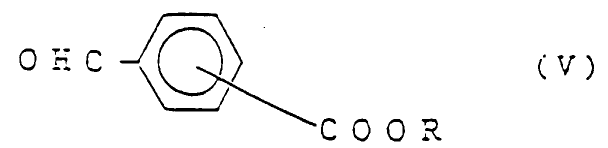 ou d'un di-(alkyl C₁-C₄)acétal de formule générale (IV): 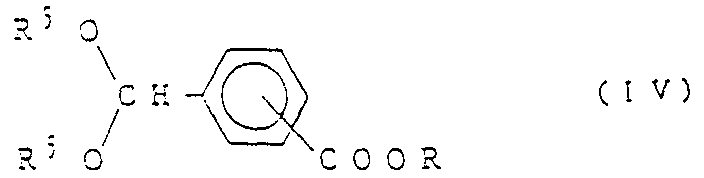 [dans laquelle R⁵ et R représentent chacun un groupe alkyle contenant de 1 à 4 atomes de carbone]
ou (a) d'un composé de formule générale (V) ou d'un composé de formule générale (IV), et (b) de benzaldéhyde ou de di(alkyl C₁-C₄)acétal de ce dernier, ou de benzaldéhyde substitué par 1 à 3 groupes alkyle C₁-C₄, ou d'un di(alkyl C₁-C₄)acétal de ce dernier, soit dans un ordre éventuel, soit sous forme de mélange, à une réaction de condensation avec un alcool polyhydrique de formule générale (II) : 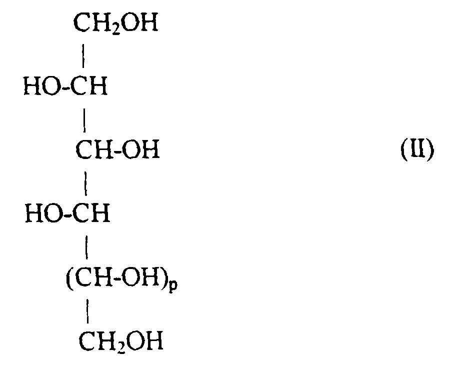 [dans laquelle p est égal à 0 ou à 1], dans un solvant organique hydrophobe et dans un solvant organique hydrosoluble, si nécessaire en présence d'un catalyseur acide,
puis la soumission du composé de formule générale (la) ainsi obtenu : 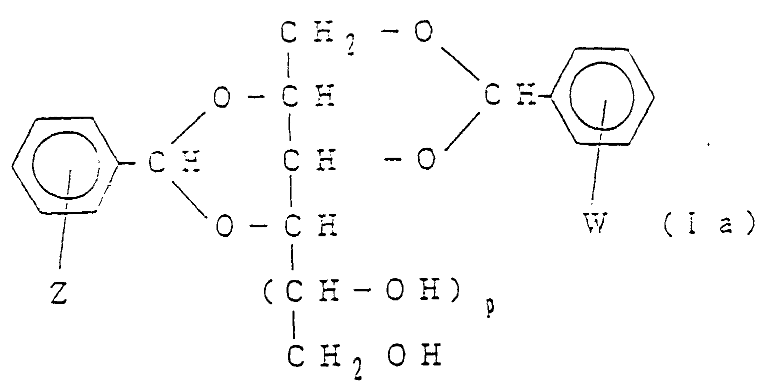 [dans laquelle W et Z sont identiques ou différents et représentent chacun un atome d'hydrogène, un groupe -(Alk)ₜ (où Alk représente un groupe alkyle C₁-C₄ et t varie de 1 à 3), ou un groupe -COOR (où R est tel que défini plus haut), à la condition que W et Z ne puissent pas être concurremment un atome d'hydrogène ou un groupe -(Alk)ₜ ; et p est égal à 0 ou à 1],
à une réaction d'échange inter-ester ou ester-amide avec au moins un alcool de formules générales :
R¹OH et R²OH
[dans lesquelles R¹ et R² sont tels que définis dans la revendication 1; à la condition que R¹ et R² soient différents de R], ou avec au moins une amine de formules générales :
R³R^{3a}NH et R⁴R^{4a}NH
[dans lesquelles R³, R^{3a}, R⁴, et R^{4a} sont tels que définis dans la revendication 1].

13. Procédé de production d'un composé de formule générale (I) selon la revendication 1, caractérisé par la soumission d'un composé de formule générale (la) : 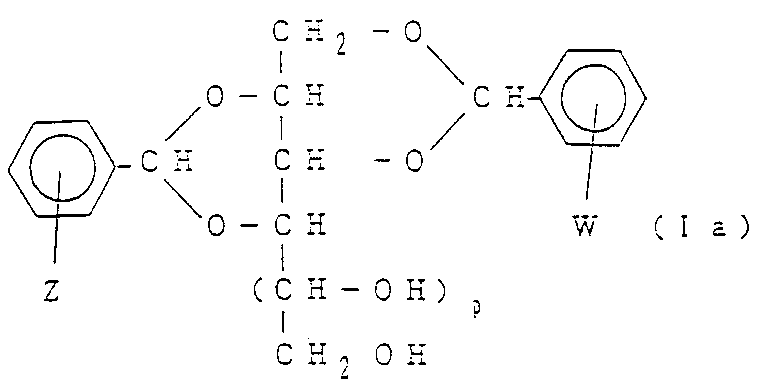 [dans laquelle W et Z sont identiques ou différents et représentent chacun un atome d'hydrogène, un groupe -(Alk)ₜ (où Alk représente un groupe alkyle C₁-C₄ et t varie de 1 à 3), ou un groupe -COOR (où R est tel que défini plus haut), à la condition que W et Z ne puissent pas être concurremment un atome d'hydrogène ou un groupe -(Alk)ₜ ; et p est égal à 0 ou à 1],
à une réaction d'échange inter-ester ou ester-amide avec au moins un alcool de formules générales :
R¹OH et R²OH
[dans lesquelles R¹ et R² sont tels que définis dans la revendication 1 ; à la condition que R¹ et R² soient différents de R], ou avec au moins une amine de formules générales :
R³R^{3a}NH et R⁴R^{4a}NH
[dans lesquelles R³, R^{3a}, R⁴, et R^{4a} sont tels que définis dans la revendication 1].

14. Procédé selon la revendication 13, dans lequel l'alcool est un alcanediol contenant de 2 à 500 atomes de carbone, un poly(alkylène C₂-C₃)glycol, un poly(degré de polymérisation = 1 à 100)oxyalkylène alkyl(C₁-C₄₀) ou alkényl(C₂-C₄₀) éther, un poly(degré de polymérisation = 1 à 100)oxyalkylène alkyl(C₁-C₄₀)phényl éther, ou un N,N-bis-[poly(degré de polymérisation = 1 à 80)oxyalkylène]alkyl-(C₁-C₃₀)amide.

15. Procédé selon la revendication 13, dans lequel l'amine est une N-(2-hydroxyéthyl)-N-alkyl(C₁-C₃₀)amine, une mono- ou une dialcanol(C₂-C₃)amine, une alkyldiamine contenant de 2 à 20 atomes de carbone, une aryldiamine contenant de 6 à 20 atomes de carbone, une aralkyldiamine contenant de 7 à 20 atomes de carbone , une polyoxyéthylènediamine (degré de polymérisation = 1 à 100), une polyoxypropylènediamine (degré de polymérisation = 1 à 100), ou une polyoxyéthylène/polyoxypropylènediamine (degré de polymérisation = 1 à 100 pour l'oxyde d'éthylène et pour l'oxyde de propylène).

16. Procédé selon la revendication 13, dans lequel l'amine est une N-(2-hydroxyéthyl)-N-alkyl(C₁-C₃₀)amine, ou une mono- ou une di-(alcanol C₂-C₃)amine.
